# FASCICULE DE BREVET EUROPEEN

(11) **EP 1 791 524 B1**
(45) Date de publication et mention de la délivrance du brevet: **19.11.2014**
(21) Numéro de dépôt: 05802489.4
(22) Date de dépôt: 15.09.2005
(51) Int. Cl.: A61K 9/10, A61K 9/20, A61K 47/36

(54) **COMPOSITION PHARMACEUTIQUE COMPRENANT UNE DISPERSION SOLIDE A MATRICE POLYMERE COMPRENANT UNE PHASE CONTINUE DE POLYDEXTROSE ET UNE PHASE CONTINUE D'UN POLYMERE AUTRE QUE DU POLYDEXTROSE**
PHARMAZEUTISCHE ZUSAMMENSETZUNG AUS EINER FESTEN DISPERSION MIT EINER POLYMERMATRIX MIT EINER KONTINUIERLICHEN POLYDEXTROSEPHASE UND EINER KONTINUIERLICHEN PHASE AUS EINEM ANDEREN POLYMER ALS POLYDEXTROSE
PHARMACEUTICAL COMPOSITION COMPRISING A SOLID DISPERSION WITH A POLYMER MATRIX CONTAINING A CONTINUOUS POLYDEXTROSE PHASE AND A CONTINUOUS PHASE OF A POLYMER OTHER THAN POLYDEXTROSE

(30) Priorité: 17.09.2004 FR 0409874
(43) Date de publication de la demande: 06.06.2007
(73) Titulaire: SANOFI, 75008 Paris (FR)
(72) Inventeur: BEDOS, Michel, 34380 VIOLS-LE-FORT (FR); BREUL, Thierry, 34110 FRONTIGNAN (FR); BYARD, STEPHEN, MORPETH, NORTHUMBERLAND NE612 XF (GB); RIBEIRO DOS SANTOS, Isabel, 31400 TOULOUSE (FR)
(74) Mandataire: Le Coupanec, Pascale A.M.P.
(86) Numéro de dépôt international: PCT/FR2005/002288
(87) Numéro de publication internationale: WO 2006/032762

(56) Documents cités:
- EP-A- 0 679 339
- DE-A1- 19 938 672
- US-A- 4 828 836
- US-A- 5 501 858
- US-A- 5 935 600
- US-A1- 2003 083 309
- US-A1- 2003 104 063

## Description

La présente invention se rapporte à une nouvelle composition pharmaceutique comprenant une dispersion solide d'au moins un principe actif dans une matrice polymère pharmaceutiquement acceptable comprenant au moins une phase continue de polydextrose et au moins une phase continue d'un polymère autre que du polydextrose.

Les compositions pharmaceutiques sous forme de dispersions solides de principes actifs sont bien connues de l'homme du métier. Elles sont généralement utilisées pour améliorer la solubilité de principes actifs, contrôler leur vitesse de libération ou améliorer leur biodisponibilité.

De nombreuses molécules de principes actifs présentent une faible biodisponibilité par la voie orale. Une faible solubilité en milieu aqueux (c'est-à-dire une solubilité dans l'eau à 25 °C inférieure à 1 mg/ml), mais également une mauvaise perméabilité, peuvent être responsables de leur faible absorption après une administration par la voie orale. De nombreuses techniques ont été mises en oeuvre afin d'améliorer la biodisponibilité par la voie orale de ces molécules. C'est le cas de la micronisation, de la formation de sels et de complexes, de la solubilisation dans des liquides, et des dispersions solides.

Le concept des dispersions solides a été introduit en 1961 par Sekiguchi et Obi (Chem. Pharm. Bull. 9, (1961), 866-872), puis repris et défini par Golderg en 1965 (J. Pharm. Sci. 54, (1965), 1145-1148) et Chiou et Riegelmann en 1971 (J. Pharm. Sci. 60(9), (1971) 1281-1302).

D'une manière générale, l'expression « dispersion solide » désigne une matrice à l'état solide, par opposition à l'état liquide ou gazeux, comprenant au moins deux constituants dont le premier, par exemple un principe actif pharmaceutique, est dispersé de la manière la plus uniforme possible au sein des autres constituants, par exemple une matrice pharmaceutiquement acceptable. Lorsque la distribution consiste en une seule phase, une telle « dispersion solide » sera plus particulièrement nommée « solution solide » : la dispersion a lieu à l'échelle moléculaire, le principe actif est alors solubilisé dans la matrice solide et se présente à l'état amorphe. Lorsque la solution solide est mise en contact avec un milieu liquide, tel que le milieu gastrique, elle peut alors facilement former une solution liquide. Lorsque la distribution ne consiste pas en une seule phase, on utilise l'expression « dispersion solide » : la dispersion se fait à l'échelle particulaire (≥ 50nm). Le principe actif y est soit complètement dispersé à l'état cristallin, soit partiellement solubilisé.

Il existe principalement deux modes de préparation de « dispersion solide » :
- la voie « solvant » basée sur la solubilisation des composants (principe actif et matrice) dans un solvant commun, suivie d'une évaporation du solvant ;
- la voie « fondue » qui consiste à faire fondre les composants (principe actif et matrice) à haute température puis à refroidir l'ensemble afin de permettre la solidification.

La voie « solvant » présente de nombreux inconvénients : une complexité de mise en oeuvre notamment une multitude d'étapes liée au traitement des solvants entraînant un coût onéreux, ainsi que des problèmes d'ordres environnementaux et de santé publique (teneurs résiduelles en solvants).

La voie « fondue » ne présente pas de tels inconvénients mais requiert l'utilisation de températures élevées pouvant affecter la stabilité chimique des principes actifs et des autres composants dé la dispersion solide.

Les dispersions solides comprises dans les compositions pharmaceutiques de l'état de la technique sont en général formées d'un principe actif dissous ou dispersé dans une matrice d'un ou plusieurs polymère(s) pharmaceutiquement acceptable(s).

Les demandes de brevets EP 0 240 904 et EP 0 240 906 (BASF AG) décrivent ainsi un procédé pour la préparation de formes pharmaceutiques solides par un procédé d'extrusion ou d'injection-moulage, utilisant préférentiellement les copolymères de la N-vinyl pyrrolidone, notamment la copovidone.

Pourtant, les compositions de l'état de la technique comprenant des dispersions solides ne permettent pas toujours d'obtenir en particulier une augmentation satisfaisante de la biodisponibilité des principes actifs peu hydrosolubles, du fait même de cette faible solubilité tout au long du tractus gastro-intestinal.

On a maintenant trouvé de manière tout à fait surprenante et inattendue qu'une nouvelle composition pharmaceutique comprenant une dispersion solide présentant une matrice polymère particulière permet d'augmenter avantageusement la biodisponibilité d'un principe actif par rapport aux dispersions solides déjà connues.

La présente invention a ainsi pour objet une composition pharmaceutique solide comprenant une dispersion solide comprenant au moins un principe actif et une matrice polymère pharmaceutiquement acceptable, caractérisée en ce que ladite matrice polymère pharmaceutiquement acceptable comprend (i) du polydextrose, sous forme d'une phase continue de polydextrose, en mélange avec (ii) au moins un polymère autre que du polydextrose, sous forme d'une phase continue de ce polymère, la proportion dudit polydextrose étant d'au moins 20 % en poids et la proportion dudit au moins un polymère autre que du polydextrose étant d'au moins 20 % en poids, par rapport au poids total de ladite matrice polymère pharmaceutiquement acceptable.

En particulier, la composition pharmaceutique selon l'invention est caractérisée en ce qu'elle est susceptible d'être obtenue par un procédé comprenant au moins une étape consistant à réaliser un mélange comprenant ledit au moins un principe actif, ledit polydextrose et ledit au moins un polymère autre que du polydextrose, dans un dispositif à vis mélangeuse et à une température de mélange comprise entre environ 50 °C et environ 250 °C.

Plus particulièrement, la présente invention a pour objet une composition pharmaceutique solide comprenant une dispersion solide comprenant au moins un principe actif et une matrice polymère pharmaceutiquement acceptable,
caractérisée en ce que ladite matrice polymère pharmaceutiquement acceptable comprend (i) du polydextrose pour favoriser la désagrégation de la composition en milieu aqueux, sous forme d'une phase continue de polydextrose, en mélange avec (ii) au moins un polymère autre que du polydextrose, sous forme d'une phase continue de ce polymère, la proportion dudit polydextrose étant d'au moins 20 % en poids et la proportion dudit au moins un polymère autre que du polydextrose étant d'au moins 20 % en poids, par rapport au poids total de ladite matrice polymère pharmaceutiquement acceptable,
et en ce que cette composition pharmaceutique est susceptible d'être obtenue par un procédé comprenant au moins une étape consistant à réaliser un mélange comprenant ledit au moins un principe actif, ledit polydextrose et ledit au moins un polymère autre que du polydextrose, dans un dispositif à vis mélangeuse et à une température de mélange comprise entre environ 50 °C et environ 250 °C.

La composition pharmaceutique selon l'invention est tout particulièrement adaptée pour une administration par voie orale.

Par « dispersion solide », on entend selon l'invention une dispersion d'au moins un principe actif dans une matrice polymère pharmaceutiquement acceptable, sous forme d'une solution solide (principe actif dispersé à l'état amorphe, solubilisé dans la matrice polymère) ou non (principe actif dispersé à l'état cristallin) ou sous une forme intermédiaire (principe actif dispersé en partie à l'état amorphe et en partie à l'état cristallin).

Par « phase continue » d'un polymère donné (polydextrose ou autre), on entend selon l'invention que ledit polymère constitue une fraction de la matrice polymère et n'est pas à l'état dispersé c'est-à-dire s'étend sans discontinuité dans les trois dimensions de la dispersion solide.

La matrice polymère pharmaceutiquement acceptable de la composition selon l'invention comprend ainsi un mélange d'au moins deux phases continues polymères, c'est-à-dire un mélange dudit polydextrose, sous forme d'une première phase continue, et dudit au moins un polymère autre que du polydextrose, sous forme d'au moins une autre phase continue, ces phases continues polymères distinctes n'étant pas discrètement dispersées l'une dans l'autre.

On a constaté que cette structure caractéristique de la composition selon l'invention résulte, d'une part, des teneurs respectives en polymères (au moins 20 % en poids, par rapport au poids total de ladite matrice polymère pharmaceutiquement acceptable) et, d'autre part, de l'étape de procédé de mélange des composants pour ladite dispersion solide par un dispositif à vis mélangeuse et à une température de mélange comprise entre environ 50 °C et environ 250 °C (effet cisaillant et plastifiant d'un mélange à cette température par une vis mélangeuse telle que celle d'un dispositif pour extrusion ou d'injection moulage), sans vouloir toutefois être lié par une quelconque théorie.

Selon la présente invention, on a constaté que le polydextrose, sous forme d'une phase continue de polydextrose d'une matrice polymère comprenant en outre au moins un polymère autre que du polydextrose, également sous forme d'une phase continue, dans une composition pharmaceutique comprenant une dispersion solide d'un principe actif dans une telle matrice polymère, notamment bicontinue, a pour fonction de favoriser la désagrégation de la composition pharmaceutique en milieu aqueux.

Par « favoriser la désagrégation de la composition pharmaceutique», on entend selon la présente invention l'accélération de la désagrégation de la dispersion solide en milieu aqueux. L'aptitude à la désagrégation est déterminée selon l'essai de désagrégation décrit à la section 2.9.1 de la Pharmacopée européenne.

Sans vouloir toutefois être lié par une quelconque théorie, la désagrégation améliorée dans l'estomac, telle que fournie par la présence de la phase continue de polydextrose dans la composition pharmaceutique selon l'invention, permettrait de réduire avantageusement les risques de concentrations locales donc de précipitation dudit principe actif en particulier dans le tractus gastro-intestinal.

Le polydextrose (n° CAS 068424044) est un polymère amorphe hydrosoluble comprenant des unités glucose liées de manière aléatoire via tous types de liaisons glucosidiques (majoritairement 1 , 6) ainsi que des teneurs minoritaires notamment en unités de glucose et de sorbitol. Une préparation du polydextrose a été décrite en particulier dans les brevets US 3 766 165 et US 3 876 794 de la société Pfizer Inc. Le polydextrose peut d'une manière générale être obtenu par un procédé comprenant une étape de réaction de condensation catalytique à partir d'un mélange comprenant du D-glucose, du sorbitol et un catalyseur acide notamment l'acide citrique ou l'acide phosphorique.

L'utilisation du polydextrose s'est d'abord développée dans le domaine alimentaire, notamment de la diététique compte tenu de sa métabolisation partielle donc de sa faible valeur calorique. Le polydextrose est par exemple mentionné dans l'ouvrage « Food Chemicals Codex » (FCC , 4ième édition, 1996).

Le polydextrose peut être avantageusement purifié, notamment par des techniques classiques de séparation sur résines échangeuses d'ions, afin d'en supprimer des produits résiduels, pour en améliorer encore les propriétés organoleptiques (acidité) et/ou de couleur (voir par exemple EP 0 458 748 ou EP 0 473 333), dans le cadre d'une utilisation alimentaire mais aussi, désormais, en vue de son utilisation dans le domaine pharmaceutique par exemple en tant qu'excipient (Pharmaceutical Excipients 2001, edited by Ray C Rowe, Paul J Sheskey and Paul J Weller, polydextrose monography, July 27, 2001).

Par « polydextrose », on entend ainsi bien entendu selon la présente invention un polydextrose pharmaceutiquement acceptable. En particulier, un polydextrose pharmaceutiquement acceptable présente de préférence une pureté d'au moins 90 % en poids de polydextrose, les composants restants comprenant en majorité des unités libres de glucose, de sorbitol, de levoglucosan (1,6 anhydro-D-glucose) et de l'eau, la pureté pouvant être déterminée par spectrophotométrie U.V. sur substance désséchée.

Le polydextrose pouvant être utilisé dans la composition selon l'invention présente de préférence un poids moléculaire d'au plus 22000 g/mol, tel que mesuré de manière connue par chromatographie par perméation de gel (ou « chromatographie d'exclusion ») équipée d'un détecteur réfractométrique.

En particulier, le polydextrose pouvant être utilisé dans la composition selon l'invention présente un poids moléculaire moyen compris entre 150 et 5000, en particulier entre 1000 et 2000.

Parmi les polydextroses pouvant être utilisés dans la composition selon l'invention, on peut citer en particulier les polydextroses commercialisés par la société Pfizer sous les dénominations « polydextrose A » et « polydextrose K », de poids moléculaire moyen compris entre 1200 et 2000, et la famille des polydextroses commercialisés par la société Danisco sous la dénomination « Litesse^{®} », tel que le « Litesse^{®} II », et plus particulièrement le « Litesse^{®} Ultra™ » de poids moléculaire moyen compris entre 182 et 5000.

Bien entendu, le terme « polydextrose » selon l'invention peut inclure un seul polydextrose donné ou un mélange de polydextroses, notamment parmi ceux mentionnés ci-dessus, pour constituer ladite phase continue de polydextrose.

Le polymère « autre que du polydextrose » compris dans la matrice polymère de la composition selon l'invention peut être tout polymère utilisable dans les dispersions solides de l'état de la technique, bien entendu un polymère autre que du polydextrose tel que défini ci-dessus.

Bien entendu, le polymère autre que du polydextrose peut comprendre un mélange de plusieurs polymères autres que du polydextrose, distincts mais miscibles entre eux.

En particulier, ledit au moins un polymère autre que du polydextrose est choisi dans le groupe constitué par :
- les polymères cellulosiques, tels que les alkylcelluloses, notamment la méthylcellulose, tels que les hydroxyalkylcelluloses, notamment l'hydroxyméthylcellulose, l'hydroxyéthylcellulose, l'hydroxypropylcellulose, l'hydroxybutylcellulose et l'hydroxypropylcellulose faiblement substituée, tels que les hydroxyalkylalkylcelluloses, notamment l'hydroxyéthylméthylcellulose et l'hydroxypropylméthylcellulose, tels que les carboxyalkylcelluloses, notamment la carboxyméthylcellulose, tels que les sels de carboxyalkylcelluloses, notamment la carboxyméthylcellulose sodium, tels que les carboxyalkylalkylcelluloses, notamment la carboxyméthyléthylcellulose, tels que les esters de dérivés de cellulose, notamment l'hydroxypropylméthylcellulose phtalate, l'hydroxypropylméthylcellulose acétate succinate et la cellulose acétate phtalate ;
- les homo- et copolymères vinyliques, tels que les polymères de la N-vinyl pyrrolidone, notamment la povidone, la copovidone et le polyvinyl alcool ;
- les polymères acryliques et méthacryliques, tels que ceux commercialisés sous la dénomination Eudragit^{®} par la société Röhm, en particulier les Eudragit^{®} E 100 et Eudragit^{®} L 100-55 ;
- les amidons chimiquement modifiés, notamment les amidons dérivés amidons extraits notamment du maïs, de la pomme de terre, du riz, du blé ou du tapioca ;
- les pectines ;
- les dérivés de la chitine tel que le chitosan ;
- les polymères d'origine naturelle tels que la gomme adragante, la gélatine, l'alginate de sodium, le pullulane, la gomme arabique, la gomme guar, l'agar-agar et la gomme xanthane ;
- les polyakylèneoxydes, tels que les polyéthylèneoxydes, les polypropylèneoxydes et les copolymères d'oxyde d'éthylène et d'oxyde de propylène,
et les mélanges de ceux-ci.

Plus particulièrement, ledit au moins un polymère autre que du polydextrose est choisi dans le groupe constitué par la méthylcellulose, l'hydroxyméthylcellulose, l'hydroxyéthylcellulose, l'hydroxypropylcellulose, l'hydroxybutylcellulose, l'hydroxypropylcellulose faiblement substituée, l'hydroxyéthylméthylcellulose, l'hydroxypropylméthylcellulose, la carboxyméthylcellulose, la carboxyméthylcellulose sodium, la carboxyméthyléthylcellulose, l'hydroxypropylméthylcellulose phtalate, l'hydroxypropylméthylcellulose acétate succinate, la cellulose acétate phtalate, la povidone, la copovidone, le polyvinyl alcool, les polymères acryliques et méthacryliques, tels que ceux commercialisés sous la dénomination Eudragit^{®} par la société Röhm, en particulier les Eudragit^{®} E 100 et Eudragit^{®} L 100-55, les amidons dérivés d'amidons extraits du maïs, de la pomme de terre, du riz, du blé ou du tapioca, les pectines, le chitosan, la gomme adragante, la gélatine, l'alginate de sodium, le pullulane, la gomme arabique, la gomme guar, l'agar-agar, la gomme xanthane, les polyéthylèneoxydes, les polypropylèneoxydes, les copolymères d'oxyde d'éthylène et d'oxyde de propylène, et les mélanges de ceux-ci.

Le polymère autre que du polydextrose pouvant être utilisé dans la composition selon l'invention est en particulier choisi dans le groupe constitué par les polymères hydrophiles autres que du polydextrose, et les mélanges de ceux-ci, et est plus particulièrement choisi dans le groupe constitué par :
- l'hydroxypropylcellulose, tel que celle commercialisée sous la dénomination Klucel^{®} par la société Aqualon;
- l'hydroxyéthylcellulose, tel que celle commercialisée sous la dénomination Natrosol^{®} par la société Aqualon ;
- les copolymères cationiques de diméthylaminoéthylméthacrylates et d'esters méthacryliques neutres, tels que ceux commercialisés sous la dénomination Eudragit^{®} E 100 par la société Röhm ;
- les copolymères anioniques d'acide méthacrylique et d'esters d'acide méthacrylique, tels que ceux commercialisés sous la dénomination Eudragit^{®} L 100-55 par la société Röhm ;
- l'hydroxypropylméthylcellulose acétate succinate, tel que celle commercialisée sous la dénomination Aqoat^{®} par la société Shin-Etsu;
- les polyéthylène glycols, de préférence ceux d'un poids moléculaire supérieur à 1500 ;
- la copovidone, c'est-à-dire le copolymère (poly(N-vinyl pyrrolidone) 60% - vinyl acétate 40%) tel que commercialisé sous la dénomination Kollidon VA 64® par la société BASF,
et les mélanges de ceux-ci.

On a pu constater en particulier que ledit au moins un polymère autre que du polydextrose, tel que défini ci-dessus, plus particulièrement lorsque ledit au moins un polymère autre que du polydextrose est un polymère hydrophile autre que du polydextrose, tel que défini ci-dessus, peut solubiliser une partie du polydextrose compris dans ladite matrice polymère pharmaceutiquement acceptable : ladite phase continue dudit au moins un polymère autre que du polydextrose peut ainsi se présenter sous forme d'une solution solide de polydextrose dans ledit au moins un polymère autre que du polydextrose, cette phase continue étant distincte de ladite phase continue de polydextrose. Ainsi, en particulier, la composition selon l'invention est caractérisée en ce que ledit au moins un polymère autre que du polydextrose, en particulier lorsque ledit au moins un polymère autre que du polydextrose est un polymère hydrophile autre que du polydextrose tel que défini ci-dessus, est sous forme d'une phase continue d'une solution solide de polydextrose dans ledit au moins un polymère autre que du polydextrose, cette phase continue étant distincte de ladite phase continue de polydextrose.

Selon un mode de réalisation particulier de la présente invention, la matrice polymère de la composition selon l'invention ne comprend que deux phases continues polymères, à savoir une première phase continue de polydextrose et une deuxième phase continue dudit au moins un polymère autre que polydextrose, la composition pharmaceutique selon l'invention présentant ainsi de manière caractéristique une matrice polymère à structure bicontinue essentiellement constituée d'une phase continue de polydextrose et d'une phase continue dudit au moins un polymère autre que du polydextrose.

L'expression «structure bicontinue » est connue pour décrire d'une manière générale une structure où d'importantes fractions de molécules de deux composés différents (ou phases différentes) forment des domaines qui s'étendent sans discontinuité dans les trois dimensions de l'espace (d'après Lindman et coll., 1989). Les structures bicontinues sont ainsi caractérisées par une surface de séparation, dite interface, traversant la totalité de l'échantillon, le divisant en deux labyrinthes distincts et contigus, s'entrelaçant. Ainsi, de chaque part de cette interface, les deux sous-volumes (ou sous-espaces) occupés par chacun des composés (ou phases) sont continus (d'après Schwarz et Gomper, 2002). Les deux labyrinthes peuvent être alors utilisés indépendamment l'un de l'autre pour traverser l'échantillon de part en part : il est ainsi possible pour chaque composé (ou phase) de relier deux points quelconques se situant au sein dudit composé (ou de ladite phase), par un chemin ne passant que par ce même composé (ou cette même phase).

L'obtention de structures solides polymères bicontinues par extrusion est décrite dans la littérature, dans des domaines autres que pharmaceutique notamment dans la demande de brevet WO 01 109 49, et dans le domaine pharmaceutique, notamment dans l'article de Dollinger et Sawan, Polymer preprints, 1990, 31 : 211-212, décrivant l'utilisation d'un mélange PLA/PE-PVAc dans lequel la phase PE-PVAc a une fonction de renfort/consolidation de la matrice.

La mise en évidence de structures bicontinues en milieu liquide par mesures de coefficients d'auto-diffusion par RMN a été extensivement décrite dans la littérature, comme par exemple dans l'article « Demonstration of bicontinuous stuctures in microemulsions using automatic-mode NMR self-diffusion measurements » de K.P. Datema et al., publié dans Magnetic resonance in chemistry Vol 30, 760-767 (1992), ou dans l'article « On the demonstration of bicontinuous structures in microemulsions » de B. Lindman et al publié dans Colloids and surfaces, 38 (1989) pages 205 à 224. Dans le cas de milieux solides formés de mélanges de polymères, plusieurs études concernant la prédictivité théorique de l'obtention de structures bi-continues ont été publiées (« Interfacial and topological measurements of bicontinuous polymer morphologies » de H. Jinnai et al. dans Physical review vol 64 010803 (2001) ou « Structuring polymer blends with bicontinuous phase morphology » de J Lyngaae-Jorgensen et al. ; dans Polymer 44 (2003) 1661-1669) ; ainsi que des résultats d'observations microscopiques ou de porosimétrie de structures de mélanges de polymères qui n'infirment pas ces prédictions (« Bicontinuous morphologies in homologous multiblock copolymers and their homopolymer blends » de J.H. Laurer et al. dans Macromolecules 1998, 31, 7546-7549 ou « Observation of fine structure in bicontinuous phase-separated domains of a polymer blend by laser scanning confocal microscopy » de H. Jinnai et al. dans Macromolecules 2001, 34 5186-5191 ou « Bicontinuous nanoporous polymers by carbon dioxide foaming » de B. Krause et al. dans macromolecules 2001, 34, 8792-8801).

Afin de mettre en évidence la continuité d'une phase solide dans un mélange solide multiphasique, il est possible d'utiliser des techniques d'analyses par résonnance magnétique nucléaire (RMN) du solide, en particulier les mesures des temps de relaxation des noyaux protons. Lors de ce type d'analyse par RMN, les noyaux atomiques sont excités à un état d'énergie supérieur à celui de leur état d'équilibre. Les noyaux excités perdent de l'énergie à travers les interactions entre les spins de noyaux adjacents (interactions spin-spin), ainsi qu'à travers les interactions avec le milieu environnant (interaction spin-réseau). La mesure de ces processus de relaxation des noyaux protons d'une molécule permet d'observer expérimentalement la mobilité moléculaire de cette molécule dans son environnement. Les deux paramètres mesurables expérimentalement par cette technique sont :
- « T1 », correspondant au temps de relaxation des protons dans un repère fixe, dont la mesure est de l'ordre de grandeur de la seconde, et qui caractérise des domaines (ou phases) d'une taille de l'ordre de 50 nm, et
- « T1p » ou « T1 Rho », correspondant au temps de relaxation des protons dans un repère tournant, dont la mesure est de l'ordre de grandeur de la milliseconde, et qui caractérise des domaines (ou phases) d'une taille comprise entre 5 nm et 50 nm.

Dans le cas d'un mélange solide de polymères, l'obtention d'une valeur unique de T1 indique que le mélange de polymères est homogène et qu'il n'est pas possible de séparer un domaine discret d'une taille supérieure à 50 nm d'une phase dispersée dans une autre : on a alors une solution solide à l'échelle de 50 nm.

De même, l'obtention d'une valeur unique de T1 Rho indique que le mélange de polymères est homogène et qu'il n'est pas possible de séparer un domaine discret d'une taille supérieure à 5 nm d'une phase dispersée dans une autre, on a alors une solution solide à l'échelle de 5 nm.

Par contre, si dans le cas d'un mélange solide de deux polymères, on obtient deux valeurs de T1 Rho, cela signifie que le mélange est composé de deux phases (ou domaines) distinctes. Dans ce dernier cas, si la valeur de T1 Rho attribuée à un polymère ne varie pas en fonction de sa concentration dans le mélange, on peut en conclure que ce polymère forme une phase continue constituée du polymère seul. Si par contre la valeur de T1 Rho attribuée à un polymère varie en fonction de sa concentration dans le mélange, on peut en conclure que ce polymère entre dans la composition d'une phase constituée des deux polymères dispersés l'un dans l'autre à une échelle comprise entre 5 nm et 50 nm, taille de l'ordre de grandeur d'une molécule de polymère, on a donc dans ce cas une solution solide d'un polymère dans l'autre.

Ainsi, selon un mode de réalisation particulier, la composition pharmaceutique selon l'invention est caractérisée en ce que ladite matrice polymère pharmaceutiquement acceptable a une structure bicontinue essentiellement constituée d'une phase continue dudit polydextrose et d'une phase continue dudit au moins un polymère autre que du polydextrose.

Plus particulièrement, la composition pharmaceutique selon l'invention est caractérisée en ce que ladite matrice polymère pharmaceutiquement acceptable a une structure bicontinue essentiellement constituée d'une phase continue dudit polydextrose et d'une phase continue dudit au moins un polymère autre que du polydextrose choisi dans le groupe constitué par les polymères hydrophiles et les mélanges de ceux-ci comme, tel que décrit ci-dessus, c'est-à-dire plus particulièrement le groupe constitué par l'hydroxypropylcellulose, l'hydroxyéthylcellulose, les copolymères cationiques de diméthylaminoéthylméthacrylates et d'esters méthacryliques neutres, les copolymères anioniques d'acide méthacrylique et d'esters d'acide méthacrylique, l'hydroxypropylméthylcellulose acétate succinate, les polyéthylène glycols, la copovidone, et les mélanges de ceux-ci.

Comme indiqué ci-dessus, on a constaté que la structure caractéristique de la composition selon l'invention résulte notamment des teneurs respectives en polymères (au moins 20 % en poids, par rapport au poids total de ladite matrice polymère pharmaceutiquement acceptable).

En particulier, la composition pharmaceutique selon l'invention est caractérisée en ce que la proportion dudit polydextrose est comprise entre environ 20 % et environ 80 % en poids, et en ce que la proportion dudit au moins un polymère autre que du polydextrose est comprise entre environ 20 % et environ 80 % en poids, par rapport au poids total de la matrice polymère pharmaceutiquement acceptable.

Selon un mode de réalisation particulier, le rapport pondéral dudit polydextrose audit au moins un polymère autre que du polydextrose, dans la matrice polymère de la composition pharmaceutique selon l'invention, est compris entre environ 20: 80 et environ 50 : 50.

La proportion de ladite matrice polymère pharmaceutiquement acceptable, dans la composition pharmaceutique selon l'invention, peut en particulier être comprise entre environ 50 % et environ 99,9 % en poids, par rapport au poids total de la composition.

Par "principe actif", on entend selon l'invention une substance médicamenteuse destinée, après administration, à provoquer une réponse préventive ou thérapeutique, ainsi qu'une association de deux ou plusieurs substances de ce type.

La composition selon l'invention peut comprendre tout principe actif connu de l'homme du métier, quelle que soit l'application thérapeutique envisagée.

Bien entendu, le principe actif doit toutefois être approprié aux conditions de ladite étape de mélange dans un dispositif à vis mélangeuse et à une température de mélange comprise entre environ 50 °C et environ 250 °C, comme décrit ci-après.

Le principe actif peut se présenter sous forme d'un sel pharmaceutiquement acceptable, solvaté ou non, ou sous forme d'un complexe, notamment avec les cyclodextrines, en particulier l'hydroxypropyl-béta-cyclodextrine.

La composition selon l'invention est plus particulièrement adaptée à l'administration d'un principe actif difficilement soluble dans l'eau. Ainsi, en particulier, ledit au moins un principe actif peut présenter une solubilité modérée en milieu aqueux, c'est-à-dire une solubilité dans l'eau inférieure à 10 mg/ml à 25 °C, une solubilité faible en milieu aqueux, c'est-à-dire une solubilité dans l'eau inférieure à 1 mg/ml à 25 °C, ou même une très faible solubilité en milieu aqueux, c'est-à-dire une solubilité dans l'eau inférieure à 0,1 mg/ml à 25 °C .

Dans la composition selon l'invention, ledit au moins un principe actif est dispersé dans ladite matrice polymère pharmaceutiquement acceptable, soit à l'état amorphe, soit à l'état cristallisé, de préférence majoritairement à l'état amorphe, la présence du principe actif sous forme amorphe favorisant en particulier sa mise en solution liquide.

Par « majoritairement à l'état amorphe », on entend selon l'invention que plus de 50 % de la masse totale dudit au moins un principe actif dispersé au sein de ladite matrice pharmaceutiquement acceptable est à l'état amorphe.

L'arrangement amorphe ou cristallin du principe actif peut être vérifié par analyse enthalpique différentielle, ou par étude de diffraction aux rayons X, mais également par des techniques de microscopie.

Comme principe actif pouvant être utilisé dans la composition selon l'invention, on peut en particulier citer :
- le N-pipéridino-5-(4-bromophényl)-1-(2,4-dichlorophényl)-4-méthylpyrazole-3-carboxamide ;
- le N-pipéridino-5-(4-chlorophényl)-1-(2,4-dichlorophényl)-4-méthylpyrazole-3-carboxamide ;
- l'amiodarone (ou 2-n-butyl-3-[3,5-diiodo-4-diéthylaminoéthoxy-benzoyl]benzofurane) et ses sels pharmaceutiquement acceptables notamment le chlorhydrate ;
- la dronédarone (ou 2-n-butyl-3-[4-(3-di-n-butylaminopropoxy)benzoyl]-5-méthylsulfonamido-benzofurane) et ses sels pharmaceutiquement acceptables notamment le chlorhydrate ;
- l'acide 2-[1-(7-chloroquinoline-4-yl)-5-(2,6-diméthoxyphenyl)-1H-pyrazole-3-carbonyl]amino-adamantane-2-carboxylique ;
- le 2-n-butyl-3-[4-[3-(dibutylamino)propyl]benzoyl]-1-benzofurane-5-carboxylate d'isopropyle et ses sels pharmaceutiquement acceptables notamment le fumarate;
- le 7-Chloro-*N,N,*5-triméthyl-4-oxo-3-phényl-3,5-dihydro-4*H*-pyridazino[4,5-b]indole-1-acétamide,
et les associations de ces principes actifs.

La proportion dudit principe actif dépend notamment de la solubilité intrinsèque du principe actif, de la dose efficace requise et du profil de dissolution souhaité.

La proportion dudit principe actif dans la composition selon l'invention peut en particulier être comprise entre environ 0,1 % et environ 50 % en poids, par rapport au poids total de la composition. Les doses équivalentes en principes actifs sont de l'ordre du milligramme au gramme, par dose unitaire.

La composition pharmaceutique selon l'invention peut comprendre en outre tout composant connu de l'homme du métier pour une composition pharmaceutique, notamment ceux pour une composition pharmaceutique comprenant une dispersion solide. En particulier, la composition pharmaceutique selon l'invention est caractérisée en ce que ledit mélange réalisé dans un dispositif à vis mélangeuse peut comprendre en outre au moins un composant choisi dans le groupe constitué par les agents plastifiants, les agents de démoulage ou lubrifiants, les agents fluidifiants, les agents antioxydants, les agents conservateurs, les colorants, les arômes, les édulcorants, les agents mouillants, les agents tampons, les agents adsorbants, les agents absorbants, les promoteurs d'absorption, en particulier la vitamine E (d-α-tocophéryl polyéthylène glycol 1000 succinate) telle que celle commercialisée sous la dénomination Eastman® Vitamin E TPGS par la société Eastman, les agents bioadhésifs, les agents désintégrants et les mélanges de ceux-ci.

Comme indiqué ci-dessus, on a constaté que la structure caractéristique de la composition selon l'invention résulte non seulement des teneurs respectives en polymères (au moins 20 % en poids, par rapport au poids total de ladite matrice polymère pharmaceutiquement acceptable) mais aussi de l'étape de procédé de mélange des composants pour ladite dispersion solide par un dispositif à vis mélangeuse et à une température de mélange comprise entre environ 50 °C et environ 250 °C (effet cisaillant et plastifiant d'un mélange à cette température, par une vis mélangeuse telle que celle d'un dispositif pour extrusion ou d'injection moulage).

Plus particulièrement, ladite température de mélange est comprise entre environ 80 °C et environ 200 °C, et plus particulièrement encore entre environ 100 °C et environ 160 °C.

La température de mélange est en particulier réglée pour être supérieure à la température de transition vitreuse du mélange, ce mélange pouvant être réalisé pendant un temps suffisant pour obtenir une plastification du mélange, une solubilisation du principe actif, et ainsi en particulier une formation desdites phases continues de polydextrose et dudit polymère autre que du polydextrose, essentiellement exemptes d'hétérogénéités. La formation de ces phases continues peut par exemple être vérifiée par des mesures de temps de relaxation du proton par RMN du solide, comme décrit ci-dessus et illustré dans les exemples ci-après.

Cette température de mélange est de préférence réglée à une température appropriée au principe actif. Elle peut par exemple être abaissée pour éviter une trop forte dégradation du principe actif, pour une matrice polymère donnée, selon des techniques connues de l'homme du métier, notamment par introduction d'au moins un agent plastifiant, pour abaisser la température de transition vitreuse de la matrice polymère, tels que le triéthyl citrate, l'éthylène glycol, le triethylène glycol, le polyéthylène glycol, le polypropylène glycol, des copolymères d'éthylène glycol et de propylène glycol, un poloxamer ou l'eau.

La température de mélange peut être obtenue en particulier par des moyens de chauffage intégrés au dispositif à vis mélangeuse.

Les composants du mélange destiné à être réalisé dans ledit dispositif à vis mélangeuse à savoir, au moins, ledit au moins un principe actif, ledit polydextrose et ledit au moins un polymère autre que du polydextrose, peuvent être introduits dans le dispositif à vis mélangeuse individuellement et/ou en mélange d'au moins une partie d'entre eux.

Par exemple, les composants peuvent être introduits sous forme d'un simple mélange préliminaire homogène ou « mélange physique », réalisé à température ambiante (environ 25°C) dans un mélangeur classique par exemple de type Turbula.

Selon un autre exemple, le polymère autre que du polydextrose peut être introduit dans ledit dispositif à vis mélangeuse sous forme d'un mélange avec au moins une partie de la quantité totale de polydextrose de la composition selon l'invention, au moins une partie de la quantité totale dudit au moins un principe actif et au moins une partie de la quantité totale d'additif(s) éventuel(s), ce mélange pouvant être en particulier sous forme d'un simple mélange physique ou sous forme d'une dispersion solide ou encore sous forme d'une solution solide, le reste des composants de la présente composition à savoir, au moins, le reste du polydextrose, le reste dudit au moins un principe actif et le reste d'additif(s) éventuel(s) étant introduits avec ce mélange dans le dispositif à vis mélangeuse pour obtenir la composition selon l'invention. De manière réciproque, bien entendu, le polydextrose peut être introduit dans ledit dispositif à vis mélangeuse sous forme d'un mélange avec au moins une partie de la quantité totale dudit polymère autre que du polydextrose, au moins une partie de la quantité totale dudit au moins un principe actif et au moins une partie de la quantité totale d'additif(s) éventuel(s), ce mélange pouvant être en particulier sous forme d'un simple mélange physique ou sous forme d'une dispersion solide ou encore sous forme d'une solution solide, le reste des composants de la présente composition à savoir, au moins, le reste dudit polymère autre que du polydextrose, le reste dudit au moins un principe actif et le reste d'additif(s) éventuel(s) étant introduits avec ce mélange dans le dispositif à vis mélangeuse pour obtenir la composition selon l'invention.

Le dispositif à vis mélangeuse peut ainsi être en particulier choisi parmi les dispositifs connus pour extrusion (extrudeuse monovis ou à plusieurs vis) ou d'injection-moulage de matières plastiques. Différentes géométries de vis peuvent être adaptées, notamment selon la composition du mélange.

Selon un mode particulier de réalisation de la présente invention, ledit dispositif à vis mélangeuse est un dispositif à bi-vis mélangeuse, dont un des avantages est notamment de fournir une plus grande force de cisaillement du mélange. La bi-vis mélangeuse peut être à fonctionnement en co-rotation ou contra-rotation.

Selon un mode de réalisation particulièrement préféré de la présente invention, ledit dispositif à vis mélangeuse est un dispositif pour extrusion, tel que, par exemple, le dispositif commercialisé sous la dénomination PolyDrive Extruder^{®} par la société Thermo Haake. Plus particulièrement, ladite étape consistant à réaliser ledit mélange dans un dispositif pour extrusion peut être avantageusement suivie d'au moins une étape consistant à mettre en forme le mélange extrudé, à la température du mélange extrudé ou après refroidissement du mélange extrudé à une température de mise en forme appropriée, choisie dans le groupe constitué par les étapes de calandrage, de filage, de découpage, et les combinaisons de ces étapes.

Selon un autre mode de réalisation particulièrement préféré de la présente invention, ledit dispositif à vis mélangeuse est un dispositif pour injection moulage, tel que, par exemple, la presse à injecter commercialisée sous la dénomination « Sprinter 11 » par la société Erinca.

Si nécessaire, l'étape de mélange dans le dispositif à vis mélangeuse peut être précédée d'une étape de mélange physique à une température appropriée, en particulier comprise entre la température ambiante (environ 25 °C) et la température du mélange dans le dispositif à vis mélangeuse, par exemple dans un mélangeur Turbula^{®}, pendant un temps suffisant (en général quelques minutes) pour obtenir un mélange physique homogène, notamment en vue de faciliter l'alimentation dudit dispositif à vis mélangeuse.

Quel que soit le dispositif à vis mélangeuse utilisé, la composition pharmaceutique selon l'invention est plus particulièrement caractérisée en ce que ledit procédé comprend en outre, après refroidissement à une température appropriée pour solidifier de manière suffisante le mélange obtenu, au moins une étape choisie dans le groupe constitué par les étapes de broyage, de découpage, et les combinaisons de ces étapes.

Bien entendu, la composition peut comprendre un enrobage tels que ceux connus de l'homme du métier notamment pour améliorer l'aspect, le goût et/ou fournir un effet de libération modifiée du principe actif.

En particulier, la désagrégation de la composition pharmaceutique favorisée en particulier par le polydextrose sous forme d'une phase continue, comme expliqué ci-dessus, n'exclue pas bien entendu une libération immédiate ou une libération modifiée (lente ou différée), soit une combinaison de ces types de libération, à partir des fragments de la composition désagrégée, en utilisant des techniques de formulation connues de l'homme du métier, notamment un enrobage pour une libération modifiée.

Ainsi, la composition pharmaceutique selon l'une quelconque des revendications précédentes peut être plus particulièrement caractérisée en ce qu'elle est susceptible d'être obtenue par un procédé comprenant en outre au moins une étape d'enrobage pour une libération modifiée.

La présente invention se rapporte également à une forme pharmaceutique solide, caractérisée en ce qu'elle comprend au moins une composition pharmaceutique telle que décrite ci-dessus, en particulier à une forme pharmaceutique solide pour une administration par voie orale.

Plus particulièrement, la présente invention a pour objet un comprimé pharmaceutique, caractérisé en ce qu'il est susceptible d'être obtenu par un procédé comprenant au moins une étape de broyage, de découpage, et les combinaisons de ces étapes, d'au moins une composition pharmaceutique, comme décrit ci-dessus, suivie d'au moins une étape de compression ou de compactage, et éventuellement d'une étape d'enrobage comme décrit également ci-dessus.

La présente invention a également pour objet une gélule pharmaceutique, caractérisée en ce qu'elle est susceptible d'être obtenue par un procédé comprenant au moins une étape de remplissage avec au moins une composition pharmaceutique, après une étape de broyage, de découpage, ou d'une combinaison de ces étapes, et éventuellement une étape d'enrobage, comme décrit ci-dessus.

La présente invention a également pour objet un comprimé pharmaceutique moulé, caractérisé en ce qu'il est constitué d'une composition pharmaceutique obtenue par un dispositif pour injection-moulage, suivie éventuellement d'une étape d'enrobage, comme décrit ci-dessus.

Enfin, la présente invention se rapporte également à l'utilisation du polydextrose pour la fabrication par extrusion ou injection moulage d'une composition pharmaceutique comprenant une dispersion solide d'au moins un principe actif dans une matrice polymère pharmaceutiquement acceptable, ladite matrice polymère comprenant du polydextrose sous forme d'une phase continue de polydextrose en mélange avec au moins un polymère autre que du polydextrose sous forme d'une phase continue de ce polymère, la proportion dudit polydextrose étant d'au moins 20 % en poids et la proportion dudit au moins un polymère autre que du polydextrose étant d'au moins 20 % en poids, par rapport au poids total de ladite matrice polymère.

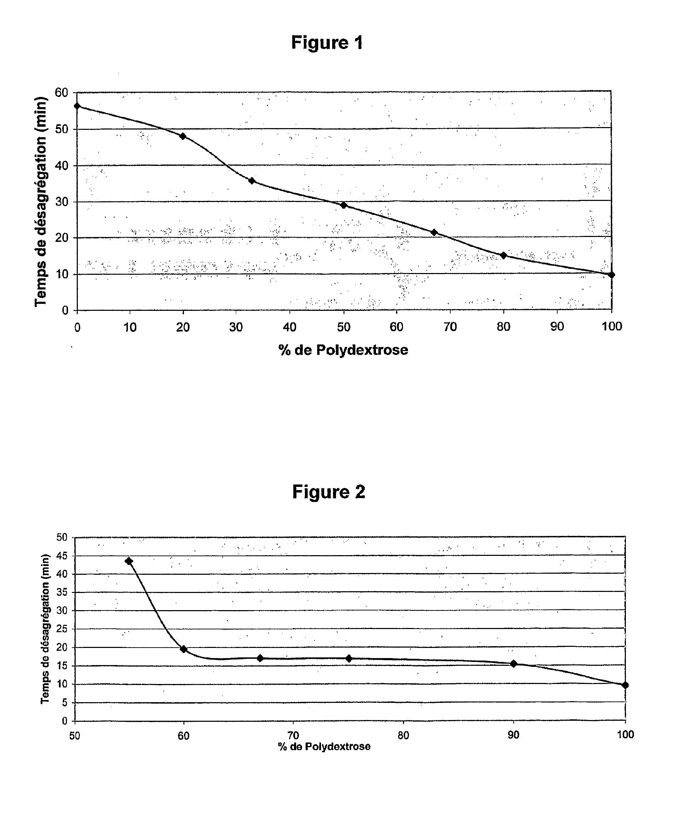

La Figure 1 est une courbe montrant le temps de désagrégation (en minutes) de comprimés moulés (préparations 1 à 7 de l'exemple 4), en fonction du pourcentage de polydextrose dans une matrice polymère polydextrose et copovidone exprimé par rapport au poids total de cette matrice polymère, selon le test de désagrégation in vitro (partie 4.1 de l'exemple 4 ; tableau 4).

La Figure 2 est une courbe montrant le temps de désagrégation (en minutes) de comprimés moulés (préparations 7 à 12), en fonction du pourcentage de polydextrose dans une matrice polymère polydextrose et Eudragit E 100 exprimé par rapport au poids total de cette matrice polymère, selon le test de désagrégation in vitro (partie 4.2 de l'exemple 4 ; tableau 5).

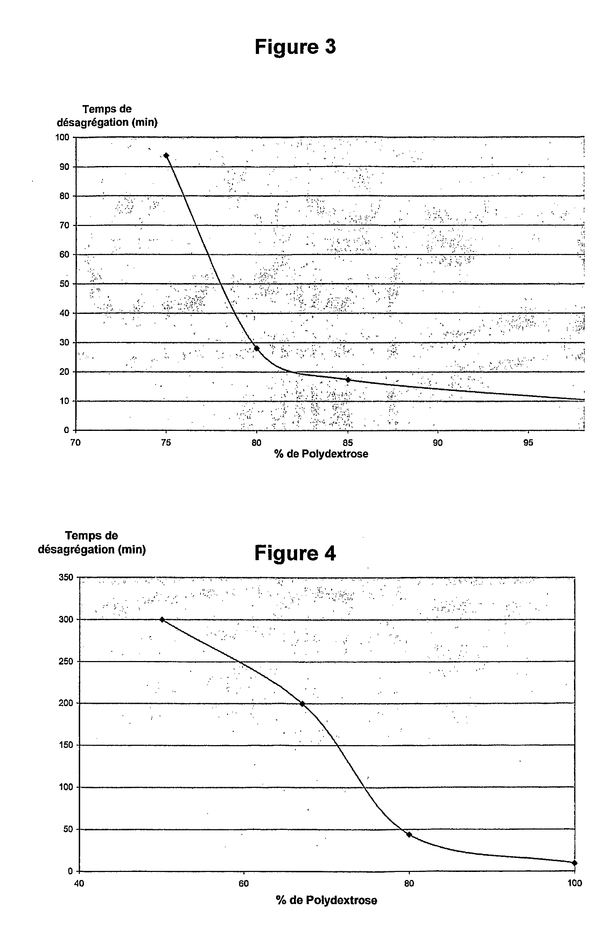

La Figure 3 est une courbe montrant le temps de désagrégation (en minutes) de comprimés moulés (préparations 7 et 13 à 15), en fonction du pourcentage de polydextrose dans une matrice polymère polydextrose et Aqoat ASMG exprimé par rapport au poids total de cette matrice polymère, selon le test de désagrégation in vitro (partie 4.3 de l'exemple 4 ; tableau 6).

La Figure 4 est une courbe montrant le temps de désagrégation (en minutes) de comprimés moulés (préparations 7 et 16 à 18), en fonction du pourcentage de polydextrose dans une matrice polymère polydextrose et Klucel EF exprimé par rapport au poids total de cette matrice polymère, selon le test de désagrégation in vitro (partie 4.4 de l'exemple 4 ; tableau 7).

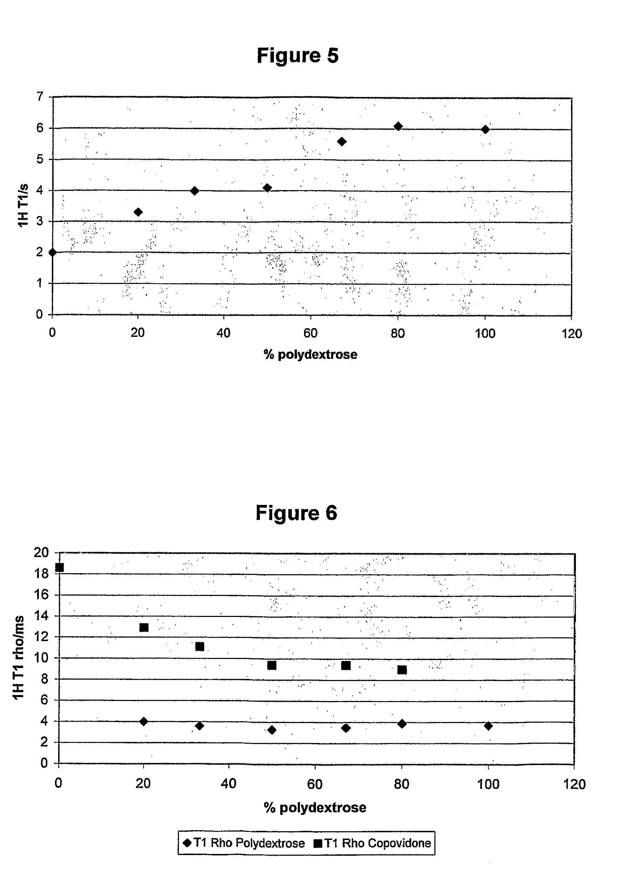

La Figure 5 est une série de points montrant les valeurs du temps de relaxation du proton (T1 ; en secondes) des comprimés moulés à matrice polydextrose et copovidone (préparations 19 et 2 à 7) comme décrit à l'exemple 5.

La Figure 6 représente deux séries de points montrant chacune les valeurs du temps de relaxation du proton (T1 Rho ; en millisecondes) des comprimés moulés à matrice polydextrose et copovidone (préparations 19 et 2 à 7) comme décrit à l'exemple 5. La série de points sous forme de losanges correspond aux valeurs des temps de relaxation (T1 Rho) du polydextrose et la série de points sous forme de carrés correspond aux valeurs des temps de relaxation (T1 Rho) de la copovidone.

Les exemples suivants sont destinés à illustrer la présente l'invention et ne doivent en aucun cas être interprétés comme pouvant en limiter la portée.

Sauf autre précision, les pourcentages en poids indiqués dans les exemples sont des pourcentages en poids exprimés par rapport au poids total.

Dans ce qui suit, on entend par « principe actif A » le principe actif N-pipéridino-5-(4-bromophényl)-1-(2,4-dichlorophényl)-4-méthylpyrazole-3-carboxamide.

### Exemple 1 : comprimé moulé à matrice polymère polydextrose et copovidone (50 : 50) et à 0,5 % de principe actif

On prépare un mélange physique comprenant 0,5 % en poids de principe actif A, 49,75 % en poids de copovidone commercialisé sous la dénomination Kollidon VA 64^{®} par la société BASF et 49,75 % en poids de polydextrose commercialisé par la société Danisco sous la dénomination Litesse Ultra^{®}. Le mélange physique est réalisé à température ambiante (environ 25 °C) à l'aide d'un mélangeur Turbula^{®}, pendant 45 minutes, pour obtenir un mélange physique homogène.

On alimente une presse à injecter de la société Erinca, modèle Sprinter 11, (injection moulage) avec ce mélange physique. Les paramètres opératoires sont les suivants :
- température de fourreau de la première zone de chauffe : 120 °C ;
- température de fourreau de la deuxième zone de chauffe : 140 °C ;
- température de la buse : 160 °C ;
- température du canal chaud : 160 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose et copovidone ainsi obtenus (rapport pondéral polydextrose : copovidone de 50 : 50) présentent une masse moyenne de 1053 mg, chaque comprimé moulé contenant une dose d'environ 5 mg de principe actif A.

### Exemple comparatif 1 : comprimé moulé à matrice polymère copovidone avec 0,5 % de principe actif

On prépare un mélange physique comprenant 0,5 % en poids de principe actif A, 99,5 % en poids de copovidone commercialisé sous la dénomination Kollidon VA 64^{®} par la société BASF. Le mélange physique est réalisé à température ambiante (environ 25 °C) à l'aide d'un mélangeur Turbula, pendant 60 minutes, pour obtenir un mélange physique homogène.

On alimente une presse à injecter de la société Erinca, modèle Sprinter 11, (injection moulage) avec ce mélange physique. Les paramètres opératoires sont les suivants :
- température de fourreau de la première zone de chauffe : 120 °C ;
- température de fourreau de la deuxième zone de chauffe : 150 °C ;
- température de la buse : 160 °C ;
- température du canal chaud : 160 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère copovidone ainsi obtenus présentent une masse moyenne de 939 mg, chaque comprimé moulé contenant une dose de 5 mg de principe actif A.

### Exemple comparatif 2 : comprimé moulé à matrice polymère polydextrose avec 0,5 % de principe actif

On prépare un mélange physique comprenant 0,5 % en poids de principe actif A, 99,5 % en poids de polydextrose commercialisé par la société Danisco sous la dénomination Litesse Ultra^{®}. Le mélange physique est réalisé à température ambiante (environ 25 °C) à l'aide d'un mélangeur Turbula^{®}, pendant 45 minutes, pour obtenir un mélange physique homogène.

On alimente une presse à injecter de la société Erinca, modèle Sprinter 11, (injection moulage) avec ce mélange physique. Les paramètres opératoires sont les suivants :
- température de fourreau de la première zone de chauffe : 120 °C ;
- température de fourreau de la deuxième zone de chauffe : 155 °C ;
- température de la buse : 160 °C ;
- température du canal chaud : 160 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose ainsi obtenus présentent une masse moyenne de 1150 mg, chaque comprimé moulé contenant une dose d'environ 5 mg de principe actif A.

### Exemple 2 : tests in vitro de dissolution

On étudie la dissolution du principe actif A, à partir des comprimés moulés préparés à l'exemple 1 et aux exemples comparatifs 1 et 2 ci-dessus.

Les cinétiques de dissolution sont mesurées dans des appareils à palette, avec un milieu de dissolution gastro-intestinal simulé à pH 6,5 composé d'un mélange de un quart en volume de milieu gastrique simulé sans enzymes selon la pharmacopée USP XXI (2 g / litre de chlorure de sodium dont le pH est ajusté à pH = 1,2 avec de l'acide chlorhydrique à 11,6 M) et de trois quarts en volume du milieu intestinal simulé sans enzymes selon la pharmacopée USP XXI (6,8 g / litre de phosphate monopotassique dont le pH est ajusté à 7,5 avec de la soude 10M), à 37°C et sous une agitation par palette à 75 tours/minutes. La concentration de principe actif A dissout dans le milieu de dissolution est déterminée en prélevant un échantillon d'1ml au temps indiqué, chaque prélèvement étant filtré sur une membrane de 5 µm puis dosé par chromatographie en phase liquide.

On effectue à chaque fois 3 mesures pour en déduire une valeur moyenne de pourcentage de dissolution pour un temps donné.

Les résultats (moyennes) sont reportés dans le tableau 1 suivant. Ils sont exprimés en pourcentage de principe actif A dissout, à raison de 2 comprimés moulés par bol contenant 500 ml de milieu de dissolution.

**Tableau 1 : dissolution du principe actif A à partir des comprimés moulés à matrice polymère copovidone et/ou polydextrose**

| Temps (min) | % Dissolution du principe actif A (moyennes de 3 mesures) | | |
|---|---|---|---|
| | Comprimés moulés de l'exemple comparatif 1 (matrice polymère copovidone) | Comprimés moulés de l'exemple comparatif 2 (matrice polymère polydextrose) | Comprimés moulés de l'exemple 1 (matrice polymère copovidone et polydextrose 50 : 50) |
| 15 | 22 | 48,67 | 37,33 |
| 30 | 40,67 | 55,50 | 62,50 |
| 60 | 66,83 | 57,50 | 86,83 |
| 120 | 84,17 | 53,33 | 90,50 |
| 180 | 84,17 | 52,17 | 90,83 |
| 240 | 84,50 | 47,50 | 89,83 |

D'après ces résultats, les comprimés moulés à matrice polymère copovidone et polydextrose (50 : 50) selon l'invention fournissent une solubilité du principe actif A, au moins à partir de 30 minutes, nettement supérieure à celle obtenue avec chacun des comprimés moulés à matrice polymère copovidone seule et des comprimés moulés à matrice polymère polydextrose seul.

### Exemple 3 : étude in vivo de biodisponibilité

On prépare encore des comprimés moulés selon l'invention, à matrice polymère polydextrose et copovidone (50 : 50) et à 0,5 % de principe actif, de la manière suivante.

On prépare un mélange physique comprenant 0,5 % en poids de principe actif A, 49,75 % en poids de copovidone commercialisé sous la dénomination Kollidon VA 64^{®} par la société BASF et 49,75 % en poids de polydextrose commercialisé par la société Danisco sous la dénomination Litesse Ultra^{®}. Le mélange physique est réalisé à température ambiante (environ 25 °C) en 2 étapes de 30 minutes à l'aide d'un mélangeur Robotainer^{®}, pour obtenir un mélange physique homogène.

On alimente une presse à injecter De la société Erinca, modèle Sprinter 11, (injection moulage) avec ce mélange physique. Les paramètres opératoires sont les suivants :
- température de fourreau de la première zone de chauffe : 120 °C ;
- température de fourreau de la deuxième zone de chauffe : 140 °C ;
- température de la buse : 160 °C ;
- température du canal chaud : 160 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose et copovidone (rapport pondéral polydextrose : copovidone de 50 : 50) ainsi obtenus présentent une masse moyenne de 1084 mg, chaque comprimé moulé contenant une dose d'environ 5 mg de principe actif A.

### Etude in vivo

On étudie la biodisponibilité d'une dose de 10 mg du principe actif A, sur 12 jeunes volontaires sains, mâles. La dose de 10 mg du principe actif A est composée de 2 comprimés moulés de 5 mg préparés comme décrit ci-dessus à cet exemple 3.

On utilise aussi en référence dans cet essai une gélule témoin fabriquée par un procédé de granulation humide de composition unitaire suivante :

**Tableau 2 : composition de la gélule témoin**

| | |
|---|---|
| Principe actif A | 5 mg |
| Amidon de maïs | 80 mg |
| Lactose monohydrate | 279 mg |
| Hypromellose | 10 mg |
| Sodium lauryl sulfate | 2 mg |
| Croscarmellose sodique | 20 mg |
| Stéarate de magnésium | 4mg |
| Gélule orange taille 0 | 1 |

La dose de 10 mg du principe actif A est composée de 2 gélules témoin de 5 mg.

L'étude comprend 4 périodes d'administrations par voie orale séparées de 7 jours. Des échantillons sanguins sont prélevés sur chaque sujet avant administration orale puis 0,5 ; 1 ; 1,5 ; 2 ; 2,5 ; 3 ; 4 ; 6 ; 8 ; 12 ; 24 ; 36 ; 48 ; 72 ; 120 et 168 heures après administration. Pour chaque prélèvement la teneur en principe actif A est déterminée par une méthode validée de LC-MS/MS avec une limite de quantification à 1 ng/ml. Pour chaque administration, la biodisponibilité du principe actif A est déterminée par la mesure de l'AUC calculée entre 0 et 120 heures après administration en ng.h/ml. Les moyennes des résultats obtenus sont reportées dans le tableau 3 suivant.

**Tableau 3 : étude in vivo de biodisponibilité**

| Forme pharmaceutique | C max (ng/ml) | T max (h) | AUC ₀₋₁₂₀ (ng.h/ml) |
|---|---|---|---|
| Comprimé moulé de l'exemple 3 | 134 | 1,5 | 1408 |
| Gélule témoin | 41 | 1,5 | 906 |

II ressort des résultats de cette étude que, à jeun, le comprimé moulé selon l'invention (exemple 3) fournit une AUC, donc une biodisponibilité du principe actif A, 1,55 fois supérieure à celle obtenue avec la gélule témoin.

### Exemple 4 : fonction du polydextrose - Tests in vitro de désagrégation

### 4.1 Matrice copovidone et polydextrose - Tests in vitro de désagrégation

On réalise les préparations 1 à 7 suivantes (sans principe actif).

### Préparation 1 : comprimé moulé à matrice polymère copovidone

On alimente une presse à injecter de la société Erinca, modèle Sprinter 11, (injection moulage) avec de la copovidone commercialisé sous la dénomination Kollidon^{®} VA 64 par la société BASF. Les paramètres opératoires sont les suivants :
- température de fourreau de la première zone de chauffe : 130 °C ;
- température de fourreau de la deuxième zone de chauffe : 139 °C ;
- température de la buse : 141 °C ;
- température du canal chaud : 170 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère copovidone ainsi obtenus présentent une masse moyenne de 988 mg.

### Préparation 2 : comprimé moulé à matrice polymère polydextrose et copovidone (20 : 80)

On prépare un mélange physique comprenant soit 80 % en poids de copovidone commercialisé sous la dénomination Kollidon^{®} VA 64 par la société BASF et 20 % en poids de polydextrose commercialisé par la société Danisco sous la dénomination Litesse Ultra^{®}. Le mélange physique est réalisé à température ambiante (environ 25°C) à l'aide d'un mélangeur Turbula^{®}, pendant 45 minutes, pour obtenir un mélange physique homogène.

On alimente une presse à injecter de la société Erinca, modèle Sprinter 11, (injection moulage) avec ce mélange. Les paramètres opératoires sont les suivants :
- température de fourreau de la première zone de chauffe : 130 °C ;
- température de fourreau de la deuxième zone de chauffe : 140 °C ;
- température de la buse : 160 °C ;
- température du canal chaud : 160 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose et copovidone (rapport pondéral polydextrose : copovidone de 20 : 80) ainsi obtenus présentent une masse moyenne de 1002 mg.

### Préparation 3 : comprimé moulé à matrice polymère polydextrose et copovidone (33 : 67)

On prépare un mélange physique comprenant soit 67 % en poids de copovidone commercialisé sous la dénomination Kollidon^{®} VA 64 par la société BASF et 33 % en poids de polydextrose commercialisé par la société Danisco sous la dénomination Litesse Ultra^{®}. Le mélange physique est réalisé à température ambiante (environ 25°C) à l'aide d'un mélangeur Turbula^{®}, pendant 35 minutes, pour obtenir un mélange physique homogène.

On alimente une presse à injecter de la société Erinca, modèle Sprinter 11, (injection moulage) avec ce mélange. Les paramètres opératoires sont les suivants :
- température de fourreau de la première zone de chauffe : 130 °C ;
- température de fourreau de la deuxième zone de chauffe : 140 °C ;
- température de la buse : 160 °C ;
- température du canal chaud : 160 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose et copovidone (rapport pondéral polydextrose : copovidone de 33 : 67) ainsi obtenus présentent une masse moyenne de 1034 mg.

### Préparation 4: comprimé moulé à matrice polymère polydextrose et copovidone (50 : 50)

On prépare un mélange physique comprenant soit 50 % en poids de copovidone commercialisé sous la dénomination Kollidon VA 64® par la société BASF et 50 % en poids de polydextrose commercialisé par la société Danisco sous la dénomination Litesse Ultra^{®}. Le mélange physique est réalisé à température ambiante (environ 25°C) à l'aide d'un mélangeur Turbula^{®}, pendant 30 minutes, pour obtenir un mélange physique homogène.

On alimente une presse à injecter de la société Erinca, modèle Sprinter 11, (injection moulage) avec ce mélange. Les paramètres opératoires sont les suivants :
- température de fourreau de la première zone de chauffe : 120 °C ;
- température de fourreau de la deuxième zone de chauffe : 140 °C ;
- température de la buse : 160 °C ;
- température du canal chaud : 160 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose et copovidone (rapport pondéral polydextrose : copovidone de 50 : 50) ainsi obtenus présentent une masse moyenne de 1117 mg.

### Préparation 5 : comprimé moulé à matrice polymère polydextrose et copovidone (67 : 33)

On prépare un mélange physique comprenant soit 33 % en poids de copovidone commercialisé sous la dénomination Kollidon VA 64® par la société BASF et 67 % en poids de polydextrose commercialisé par la société Danisco sous la dénomination Litesse Ultra^{®}. Le mélange physique est réalisé à température ambiante (environ 25°C) à l'aide d'un mélangeur Turbula, pendant 40 minutes, pour obtenir un mélange physique homogène.

On alimente une presse à injecter de la société Erinca, modèle Sprinter 11, (injection moulage) avec ce mélange. Les paramètres opératoires sont les suivants :
- température de fourreau de la première zone de chauffe : 130 °C ;
- température de fourreau de la deuxième zone de chauffe : 140 °C ;
- température de la buse : 160 °C ;
- température du canal chaud : 160 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose et copovidone (rapport pondéral polydextrose : copovidone de 67 : 33) ainsi obtenus présentent une masse moyenne de 1099 mg.

### Préparation 6: comprimé moulé à matrice polymère polydextrose et copovidone (80 : 20)

On prépare un mélange physique comprenant soit 20 % en poids de copovidone commercialisé sous la dénomination Kollidon VA 64® par la société BASF et 80 % en poids de polydextrose commercialisé par la société Danisco sous la dénomination Litesse Ultra^{®}. Le mélange physique est réalisé à température ambiante (environ 25°C) à l'aide d'un mélangeur Turbula, pendant 120 minutes, pour obtenir un mélange physique homogène.

On alimente une presse à injecter de la société Erinca, modèle Sprinter 11, (injection moulage) avec ce mélange. Les paramètres opératoires sont les suivants :
- température de fourreau de la première zone de chauffe : 130 °C ;
- température de fourreau de la deuxième zone de chauffe : 140 °C ;
- température de la buse : 160 °C ;
- température du canal chaud : 160 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose et copovidone (rapport pondéral polydextrose : copovidone de 80 : 20) ainsi obtenus présentent une masse moyenne de 1144 mg.

### Préparation 7 : comprimé moulé à matrice polymère polydextrose

On alimente une presse à injecter de la société Erinca, modèle Sprinter 11, (injection moulage) avec du polydextrose commercialisé par la société Danisco sous la dénomination Litesse Ultra^{®}. Les paramètres opératoires sont les suivants :
- température de fourreau de la première zone de chauffe : 120 °C ;
- température de fourreau de la deuxième zone de chauffe : 145 °C ;
- température de la buse : 150 °C ;
- température du canal chaud : 160 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose ainsi obtenus présentent une masse moyenne de 1186 mg.

### Test in vitro de désagrégation des comprimés moulés à matrice copovidone et polydextrose

On étudie l'aptitude à la désagrégation des comprimés moulés à matrice polymère polydextrose et/ou copovidone des préparations 1 à 7 ci-dessus, dans de l'eau déminéralisée comme milieu de désagrégation à une température de 37 +/- 2 °C, selon les conditions du test de désintégration des comprimés tel que décrit à la section 2.9.1. de la Pharmacopée européenne.

On effectue à chaque fois 3 mesures pour en déduire une valeur moyenne de temps de désagrégation en minutes.

Les résultats sont reportés dans le tableau 4 ci-après et font l'objet de la Figure 1.

**Tableau 4 : test in vitro de désagrégation des comprimés moulés à matrice copovidone et polydextrose**

| Comprimés Moulés | % copovidone de la matrice polymère | % polydextrose de la matrice polymère | Temps de désagrégation (moyenne ; en minutes) |
|---|---|---|---|
| Préparation 1 | 100 | 0 | 56,4 |
| Préparation 2 | 80 | 20 | 48 |
| Préparation 3 | 67 | 33 | 35,7 |
| Préparation 4 | 50 | 50 | 29 |
| Préparation 5 | 33 | 67 | 21,3 |
| Préparation 6 | 20 | 80 | 15 |
| Préparation 7 | 0 | 100 | 9,5 |

D'après ces résultats, l'ajout de polydextrose à la copovidone, sous forme d'un mélange de deux phases continues respectives de ces deux polymères (non discrètement dispersés l'un dans l'autre) permet de diminuer le temps de désagrégation des comprimés moulés.

### 4.2 Matrice Eudragit^{®} E100 et polydextrose - Test in vitro de désagrégation

On réalise les préparations 8 à 12 suivantes (sans principe actif).

### Préparation 8 : comprimé moulé à matrice polymère polydextrose et Eudragit^{®} E100 (55 : 45)

On prépare un mélange physique comprenant soit 45 % en poids de polymère acrylique et méthacrylique commercialisé sous la dénomination Eudragit^{®} E 100 par la société Röhm, et 55 % en poids de polydextrose commercialisé par la société Danisco sous la dénomination Litesse Ultra^{®}. Le mélange physique est réalisé à température ambiante (environ 25°C) à l'aide d'un mélangeur Turbula^{®}, pendant 30 minutes, pour obtenir un mélange physique homogène.

On alimente une presse à injecter de la société Erinca, modèle Sprinter 11, (injection moulage) avec ce mélange. Les paramètres opératoires sont les suivants :
- température de fourreau de la première zone de chauffe : 140 °C ;
- température de fourreau de la deuxième zone de chauffe : 150 °C ;
- température de la buse : 160 °C ;
- température du canal chaud : 180 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose et Eudragit^{®} E 100 (rapport pondéral polydextrose : Eudragit^{®} E 100 de 55 : 45) ainsi obtenus présentent une masse moyenne de 1015 mg.

### Préparation 9 : comprimé moulé à matrice polymère polydextrose et Eudragit^{®} E100 (60 : 40)

On prépare un mélange physique comprenant soit 40 % en poids de polymère acrylique et méthacrylique commercialisé sous la dénomination Eudragit^{®} E 100 par la société Röhm, et 60 % en poids de polydextrose commercialisé par la société Danisco sous la dénomination Litesse Ultra^{®}. Le mélange physique est réalisé à température ambiante (environ 25°C) à l'aide d'un mélangeur Turbula^{®}, pendant 30 minutes, pour obtenir un mélange physique homogène.

On alimente une presse à injecter de la société Erinca, modèle Sprinter 11, (injection moulage) avec ce mélange. Les paramètres opératoires sont les suivants :
- température de fourreau de la première zone de chauffe : 140 °C ;
- température de fourreau de la deuxième zone de chauffe : 150 °C ;
- température de la buse : 160 °C ;
- température du canal chaud : 180 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose et Eudragit^{®} E 100 (rapport pondéral polydextrose : Eudragit^{®} E 100 de 60 : 40) ainsi obtenus présentent une masse moyenne de 1048 mg.

### Préparation 10 : comprimé moulé à matrice polymère polydextrose et Eudragit^{®} E100 (67 : 33)

On prépare un mélange physique comprenant soit 33 % en poids de polymère acrylique et méthacrylique commercialisé sous la dénomination Eudragit^{®} E 100 par la société Röhm, et 67 % en poids de polydextrose commercialisé par la société Danisco sous la dénomination Litesse Ultra^{®}. Le mélange physique est réalisé à température ambiante (environ 25°C) à l'aide d'un mélangeur Turbula^{®}, pendant 60 minutes, pour obtenir un mélange physique homogène.

On alimente une presse à injecter de la société Erinca, modèle Sprinter 11, (injection moulage) avec ce mélange. Les paramètres opératoires sont les suivants :
- température de fourreau de la première zone de chauffe : 140 °C ;
- température de fourreau de la deuxième zone de chauffe : 150 °C ;
- température de la buse : 160 °C ;
- température du canal chaud : 180 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose et Eudragit^{®} E 100 (rapport pondéral polydextrose : Eudragit^{®} E 100 de 67 : 33) ainsi obtenus présentent une masse moyenne de 1057 mg.

### Préparation 11 : comprimé moulé à matrice polymère polydextrose et Eudragit^{®} E100 (75 : 25)

On prépare un mélange physique comprenant soit 25 % en poids de polymère acrylique et méthacrylique commercialisé sous la dénomination Eudragit^{®} E 100 par la société Röhm, et 75 % en poids de polydextrose commercialisé par la société Danisco sous la dénomination Litesse Ultra^{®}. Le mélange physique est réalisé à température ambiante (environ 25°C) à l'aide d'un mélangeur Turbula^{®}, pendant 30 minutes, pour obtenir un mélange physique homogène.

On alimente une presse à injecter de la société Erinca, modèle Sprinter 11, (injection moulage) avec ce mélange. Les paramètres opératoires sont les suivants :
- température de fourreau de la première zone de chauffe : 130 °C ;
- température de fourreau de la deuxième zone de chauffe : 140 °C ;
- température de la buse : 160 °C ;
- température du canal chaud : 180 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose et Eudragit^{®} E 100 (rapport pondéral polydextrose : Eudragit^{®} E 100 de 75 : 25) ainsi obtenus présentent une masse moyenne de 1119 mg.

### Préparation 12 : comprimé moulé à matrice polymère polydextrose et Eudragit^{®} E100 (90 : 10)

On prépare un mélange physique comprenant soit 10 % en poids de polymère acrylique et méthacrylique commercialisé sous la dénomination Eudragit^{®} E 100 par la société Röhm, et 90 % en poids de polydextrose commercialisé par la société Danisco sous la dénomination Litesse Ultra^{®}. Le mélange physique est réalisé à température ambiante (environ 25°C) à l'aide d'un mélangeur Turbula^{®}, pendant 30 minutes, pour obtenir un mélange physique homogène.

On alimente une presse à injecter de la société Erinca, modèle Sprinter 11, (injection moulage) avec ce mélange. Les paramètres opératoires sont les suivants :
- température de fourreau de la première zone de chauffe : 130 °C ;
- température de fourreau de la deuxième zone de chauffe : 140 °C ;
- température de la buse : 160 °C ;
- température du canal chaud : 180 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose et Eudragit^{®} E 100 (rapport pondéral polydextrose : Eudragit^{®} E 100 de 90 : 10) ainsi obtenus présentent une masse moyenne de 1188 mg.

### Test in vitro de désagrégation des comprimés moulés à matrice Eudragit® E 100 et polydextrose

On étudie l'aptitude à la désagrégation des comprimés moulés à matrice polymère polydextrose et/ou Eudragit^{®} E 100 des préparations 7 à 12 ci-dessus, dans de l'eau déminéralisée comme milieu de désagrégation à une température de 37 ± 2 °C, selon les conditions du test de désintégration des comprimés tel que décrit à la section 2.9.1. de la Pharmacopée européenne.

On effectue à chaque fois 3 mesures pour en déduire une valeur moyenne de temps de désagrégation en minutes.

Les résultats sont reportés dans le tableau 5 ci-après et font l'objet de la Figure 2.

**Tableau 5 : test in vitro de désagrégation des comprimés moulés à matrice Eudragit^{®} E 100 et polydextrose**

| Comprimés moulés | % Eudragit^{®} E 100 de la matrice polymère | % polydextrose de la matrice polymère | Temps de désagrégation (moyenne ; en minutes) |
|---|---|---|---|
| Préparation 8 | 45 | 55 | 43,6 |
| Préparation 9 | 40 | 60 | 19,6 |
| Préparation 10 | 33 | 67 | 17,1 |
| Préparation 11 | 25 | 75 | 16,9 |
| Préparation 12 | 10 | 90 | 15,4 |
| Préparation 7 | 0 | 100 | 9,5 |

D'après ces résultats, l'ajout de polydextrose à l'Eudragit^{®} E 100, sous forme d'un mélange de deux phases continues respectives de ces deux polymères (non discrètement dispersés l'un dans l'autre) permet de diminuer le temps de désagrégation des comprimés moulés.

### 4.3 Matrice Aqoat^{®} ASMG et polydextrose - Test in vitro de désagrégation

On réalise les préparations 13 à 15 suivantes (sans principe actif).

### Préparation 13 : comprimé moulé à matrice polymère polydextrose et Aqoat^{®} ASMG (75 : 25)

On prépare un mélange physique comprenant soit 25 % en poids d'hydroxypropylméthylcellulose acétate succinate, commercialisé sous la dénomination Aqoat^{®} ASMG par la société Shin-Etsu, et 75 % en poids de polydextrose commercialisé par la société Danisco sous la dénomination Litesse Ultra^{®}. Le mélange physique est réalisé à température ambiante (environ 25°C) à l'aide d'un mélangeur Turbula^{®}, pendant 40 minutes, pour obtenir un mélange physique homogène.

On alimente une presse à injecter de la société Erinca, modèle Sprinter 11, (injection moulage) avec ce mélange. Les paramètres opératoires sont les suivants :
- température de fourreau de la première zone de chauffe : 125 °C ;
- température de fourreau de la deuxième zone de chauffe : 145 °C ;
- température de la buse : 160 °C ;
- température du canal chaud : 170 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose et Aqoat^{®} ASMG (rapport pondéral polydextrose : Aqoat^{®} ASMG de 75 : 25) ainsi obtenus présentent une masse moyenne de 1182 mg.

### Préparation 14 : comprimé moulé à matrice polymère polydextrose et Aqoat^{®} ASMG (80 : 20)

On prépare un mélange physique comprenant soit 20 % en poids d'hydroxypropylméthylcellulose acétate succinate, commercialisé sous la dénomination Aqoat^{®} ASMG par la société Shin-Etsu, et 80 % en poids de polydextrose commercialisé par la société Danisco sous la dénomination Litesse Ultra^{®}. Le mélange physique est réalisé à température ambiante (environ 25°C) à l'aide d'un mélangeur Turbula^{®}, pendant 40 minutes, pour obtenir un mélange physique homogène.

On alimente une presse à injecter de la société Erinca, modèle Sprinter 11, (injection moulage) avec ce mélange. Les paramètres opératoires sont les suivants :
- température de fourreau de la première zone de chauffe : 130 °C ;
- température de fourreau de la deuxième zone de chauffe : 140 °C ;
- température de la buse : 145 °C ;
- température du canal chaud : 150 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose et Aqoat^{®} ASMG (rapport pondéral polydextrose : Aqoat^{®} ASMG de 80 : 20) ainsi obtenus présentent une masse moyenne de 1101 mg.

### Préparation 15 : comprimé moulé à matrice polymère polydextrose et Aqoat^{®} ASMG (85 : 15)

On prépare un mélange physique comprenant soit 15 % en poids d'hydroxypropylméthylcellulose acétate succinate, commercialisé sous la dénomination Aqoat^{®} ASMG par la société Shin-Etsu, et 85 % en poids de polydextrose commercialisé par la société Danisco sous la dénomination Litesse Ultra^{®}. Le mélange physique est réalisé à température ambiante (environ 25°C) à l'aide d'un mélangeur Turbula^{®}, pendant 40 minutes, pour obtenir un mélange physique homogène.

On alimente une presse à injecter de la société Erinca, modèle Sprinter 11, (injection moulage) avec ce mélange. Les paramètres opératoires sont les suivants :
- température de fourreau de la première zone de chauffe : 130 °C ;
- température de fourreau de la deuxième zone de chauffe : 140 °C ;
- température de la buse : 145 °C ;
- température du canal chaud : 150 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose et Aqoat^{®} ASMG (rapport pondéral polydextrose : Aqoat^{®} ASMG de 85 : 15) ainsi obtenus présentent une masse moyenne de 1122 mg.

### Test in vitro de désagrégation des comprimés moulés à matrice hydroxypropylméthylcellulose acétate succinate (Agoat^{®} ASMG) et polydextrose

On étudie l'aptitude à la désagrégation des comprimés moulés à matrice polymère polydextrose et/ou Aqoat^{®} ASMG des préparations 7 et 13 à 15 ci-dessus, dans de l'eau déminéralisée comme milieu de désagrégation à une température de 37 ± 2 °C, selon les conditions du test de désintégration des comprimés tel que décrit à la section 2.9.1. de la Pharmacopée européenne.

On effectue à chaque fois 3 mesures pour en déduire une valeur moyenne de temps de désagrégation en minutes.

Les résultats sont reportés dans le tableau 6 ci-après et font l'objet de la Figure 3.

**Tableau 6 : Test in vitro de désagrégation des comprimés moulés à matrice hydroxypropylméthylcellulose acétate succinate et polydextrose**

| Comprimés moulés | % Aqoat^{®} ASMG de la matrice polymère | % polydextrose de la matrice polymère | Temps de désagrégation (moyenne ; en minutes) |
|---|---|---|---|
| Préparation 13 | 25 | 75 | 93,8 |
| Préparation 14 | 20 | 80 | 28 |
| Préparation 15 | 15 | 85 | 17,4 |
| Préparation 7 | 0 | 100 | 9,5 |

D'après ces résultats, l'ajout de polydextrose à l'Aqoat^{®} ASMG, sous forme d'un mélange de deux phases continues respectives de ces deux polymères (non discrètement dispersés l'un dans l'autre) permet de diminuer le temps de désagrégation des comprimés moulés.

### 4.4 Matrice hydroxypropylcellulose et polydextrose

On réalise les préparations 16 à 18 suivantes (sans principe actif).

### Préparation 16 : comprimé moulé à matrice polymère polydextrose et hydroxypropylcellulose (50: 50 ; avec 2% en poids de vitamine E polyéthylène glycol succinate)

On prépare un mélange physique comprenant soit 49 % en poids l'hydroxypropylcellulose, tel que celle commercialisée sous la dénomination Klucel^{®}EF par la société Aqualon; 49 % en poids de polydextrose commercialisé par la société Danisco sous la dénomination Litesse Ultra^{®} ; et 2% en poids de vitamine E polyéthylène glycol succinate telle que commercialisée par la société Eastman sous la dénomination Eastman^{®} Vitamin E TPGS. Le mélange physique est réalisé à une température d'environ 50°C à l'aide d'un mélangeur Rayneri à pale en hélice inversée, pendant environ 15 minutes, pour obtenir un mélange physique homogène.

On alimente une presse à injecter de la société Erinca, modèle Sprinter 11, (injection moulage) avec ce mélange. Les paramètres opératoires sont les suivants :
- température de fourreau de la première zone de chauffe : 125 °C ;
- température de fourreau de la deuxième zone de chauffe : 146 °C ;
- température de la buse : 155 °C ;
- température du canal chaud : 160 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose, hydroxypropylcellulose et vitamine E polyéthylene glycol succinate (rapport pondéral polydextrose : hydroxypropylcellulose de 50 : 50) ainsi obtenus présentent une masse moyenne de 1051 mg.

### Préparation 17 : comprimé moulé à matrice polymère polydextrose et hydroxypropylcellulose (67 : 33 ; avec 2 % en poids de vitamine E polyéthylène glycol succinate)

On prépare un mélange physique comprenant soit 32,3 % en poids d'hydroxypropylcellulose, tel que celle commercialisée sous la dénomination Klucel^{®}EF par la société Aqualon; 65,7 % en poids de polydextrose commercialisé par la société Danisco sous la dénomination Litesse Ultra^{®}; et 2% de vitamine E polyéthylène glycol succinate telle que commercialisée par la société Eastman sous la dénomination Eastman^{®} Vitamin E TPGS. Le mélange physique est réalisé à une température d'environ 50°C à l'aide d'un mélangeur Rayneri à pale en hélice inversée, pendant environ 15 minutes, pour obtenir un mélange physique homogène.

On alimente une presse à injecter de la société Erinca, modèle Sprinter 11, (injection moulage) avec ce mélange. Les paramètres opératoires sont les suivants :
- température de fourreau de la première zone de chauffe : 130 °C ;
- température de fourreau de la deuxième zone de chauffe : 140 °C ;
- température de la buse : 150 °C ;
- température du canal chaud : 150 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose, hydroxypropylcellulose et vitamine E polyéthylène glycol succinate (rapport pondéral polydextrose : hydroxypropylcellulose de 67 : 33) ainsi obtenus présentent une masse moyenne de 1126 mg.

### Préparation 18 : comprimé moulé à matrice polymère polydextrose et hydroxypropylcellulose (80: 20 ; avec 2% de vitamine E polyéthylène glycol succinate)

On prépare un mélange physique comprenant soit 19,6 % en poids l'hydroxypropylcellulose, tel que celle commercialisée sous la dénomination Klucel^{®}EF par la société Aqualon; 78,4 % en poids de polydextrose commercialisé par la société Danisco sous la dénomination Litesse Ultra^{®} ; et 2% de vitamine E polyéthylène glycol succinate telle que commercialisée par la société Eastman sous la dénomination Eastman^{®} Vitamin E TPGS. Le mélange physique est réalisé à une température d'environ 50°C à l'aide d'un mélangeur Rayneri à pale en hélice inversée, pendant environ 15 minutes, pour obtenir un mélange physique homogène.

On alimente une presse à injecter de la société Erinca, modèle Sprinter 11, (injection moulage) avec ce mélange. Les paramètres opératoires sont les suivants :
- température de fourreau de la première zone de chauffe : 130 °C ;
- température de fourreau de la deuxième zone de chauffe : 140 °C ;
- température de la buse : 150 °C ;
- température du canal chaud : 150 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose, hydroxypropylcellulose et vitamine E polyéthylène glycol succinate (rapport pondéral polydextrose : hydroxypropylcellulose de 80 : 20) ainsi obtenus présentent une masse moyenne de 1146 mg.

### Tests in vitro de désagrégation des comprimés moulés à matrice hydroxypropylcellulose (Klucel^{®} EF) et polydextrose

On étudie l'aptitude à la désagrégation des comprimés moulés à matrice polymère polydextrose et/ou Klucel^{®} EF des préparations 7 et 16 à 18 ci-dessus, dans de l'eau déminéralisée comme milieu de désagrégation à une température de 37 ± 2 °C, selon les conditions du test de désintégration des comprimés tel que décrit à la section 2.9.1. de la Pharmacopée européenne.

On effectue à chaque fois 3 mesures pour en déduire une valeur moyenne de temps de désagrégation en minutes.

Les résultats sont reportés dans le tableau 7 ci-après et font l'objet de la Figure 4.

**Tableau 7 : test in vitro de désagrégation des comprimés moulés à matrice hydroxypropylcellulose et polydextrose²**

| Comprimés Moulés | % Klucel^{®} EF de la matrice polymère | % polydextrose de la matrice polymère | Temps de désagrégation (moyennes ; en minutes) |
|---|---|---|---|
| Préparation 16 | 50 | 50 | 300 |
| Préparation 17 | 33 | 67 | 200 |
| Préparation 18 | 20 | 80 | 44 |
| Préparation 7 | 0 | 100 | 9,5 |

D'après ces résultats, l'ajout de polydextrose à l'hydroxypropylcellulose (Klucel^{®} EF), sous forme d'un mélange de deux phases continues respectives de ces deux polymères (non discrètement dispersés l'un dans l'autre) permet de diminuer le temps de désagrégation des comprimés moulés.

### Exemple 5 : analyses des temps de relaxation du proton par RMN du solide des comprimés moulés à matrice polydextrose et copovidone

On réalise encore la préparation 19 suivante (sans principe actif).

### Préparation 19 : comprimé moulé à matrice polymère copovidone

On alimente une presse à injecter de la société Erinca, modèle Sprinter 11, (injection moulage) avec de la copovidone commercialisé sous la dénomination Kollidon VA 64^{®} par la société BASF. Les paramètres opératoires sont les suivants :
- température de fourreau de la première zone de chauffe : 120 °C ;
- température de fourreau de la deuxième zone de chauffe : 160 °C ;
- température de la buse : 160 °C ;
- température du canal chaud : 170 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère copovidone ainsi obtenus présentent une masse moyenne de 972 mg.

### Analyses par RMN du solide

Les mesures des temps de relaxation du proton T1 et T1 Rho par RMN du solide, des comprimés moulés à matrice copovidone et/ou polydextrose des préparations 2 à 7 et 19 ci-dessus sont reportées dans le tableau 8 ci-après. Ces résultats font l'objet de la figure 5 pour la représentation de T1 en secondes, en fonction du pourcentage de polydextrose contenu dans la matrice, et de la figure 6 pour la représentation de T1 Rho en millisecondes, en fonction du pourcentage de polydextrose contenu dans la matrice.

Les mesures des temps de relaxation du proton T1 et T1 Rho par RMN du solide, ont été effectuées selon la méthode décrite dans l'article « Investigation of the physical stability of amorphous drug and drug polymer melts using variable temperature solid state NMR » de A. Forster et al. publié dans Pharmazie Vol 58 (2003) pages 761 à 762 ; mais à température constante et en faisant varier les taux respectifs de polydextrose et de copovidone dans la matrice polymérique. Chacune des valeurs décrites est la moyenne des résultats obtenus après 3 essais.

D'après ces résultats (voir Figure 5), pour chaque mélange polydextrose et copovidone, on obtient une seule valeur de relaxation du proton : T₁ (exprimée en secondes), ce qui signifie qu'on ne peut différencier les phases copovidone et polydextrose en présence, à l'échelle de 50 nm (sensibilité de la mesure). En d'autres termes, on ne peut distinguer de domaine discret, d'une phase dispersée dans l'autre, dont la taille serait supérieure à 50 nm. Aucune des deux phases polydextrose et copovidone n'est donc discontinue à cette échelle, qui correspond à une échelle macro-moléculaire pour ces deux polymères. On constate aussi que la valeur de T1 varie avec la composition de la matrice et reflète la proportion de polydextrose présente dans la matrice.

**Tableau 8 : valeurs des temps relaxation du proton (T1 en secondes et T1 Rho en millisecondes) des comprimés moulés à matrice polydextrose et copovidone**

| Comprimés moulés | % polydextrose de la matrice polymère | % copovidone de la matrice polymère | T1 (s) | T1 Rho polydextrose (ms) | T1 Rho copovidone (ms) |
|---|---|---|---|---|---|
| Préparation 19 | 0 | 100 | 2,0 | - | 18,6 |
| Préparation 2 | 20 | 80 | 3,3 | 4,0 | 12,9 |
| Préparation 3 | 33 | 67 | 4,0 | 3,6 | 11,1 |
| Préparation 4 | 50 | 50 | 4,1 | 3,3 | 9,4 |
| Préparation 5 | 67 | 33 | 5,6 | 3,5 | 9,4 |
| Préparation 6 | 80 | 20 | 6,1 | 3,9 | 9 |
| Préparation 7 | 100 | 0 | 6,0 | 3,7 | - |

D'après ces résultats (voir Figure 6), pour chaque mélange polydextrose et copovidone, on obtient deux valeurs de relaxation du proton : T₁ Rho (exprimées en millisecondes), ce qui signifie qu'on peut différencier deux phases distinctes à une échelle de mesure comprise entre 5 nm et 50 nm, qui correspond à l'échelle moléculaire pour le polydextrose et la copovidone. Ces résultats nous indiquent aussi la composition des deux phases observées. Le T1 Rho attribué au polydextrose reste sensiblement constant indépendamment de la composition de la matrice analysée, ce qui indique qu'une des phases est constituée exclusivement de polydextrose. Le T1 Rho attribué à la copovidone varie avec la composition de la matrice analysée jusqu'à ce que la matrice contienne 50 % de polydextrose, puis reste constant pour des proportions de polydextrose supérieures à 50 %. Cela signifie que la phase copovidone solubilise du polydextrose jusqu'à saturation (50 % de polydextrose). A l'échelle moléculaire de ces deux polymères mélangés et fondus, coexistent donc deux phases polymères, l'une constituée de polydextrose seul, l'autre phase polymère étant une solution solide de polydextrose dans la copovidone.

Ces résultats prouvent donc que :
- à l'échelle macro-moléculaire, Il n'existe pas de phase discrète d'un polymère dispersée dans une autre (T1 a une valeur unique), la matrice est donc continue.
- les comprimés moulés à matrice polydextrose et copovidone sont constitués de deux phases polymères distinctes à l'échelle moléculaire, une phase de polydextrose seul et une phase polymère constituée par une solution solide de polydextrose dans la copovidone.
- à l'échelle moléculaire, la phase constituée du polydextrose seul est continue (invariance du T1 Rho attribué au polydextrose, en fonction de la composition de la matrice).

En conclusion, les comprimés moulés à matrices constituées d'un mélange de polydextrose et de copovidone fondus ne contiennent pas de structures discontinues et ils sont constitués de deux phases polymères distinctes dont une (la phase polydextrose seul) est continue. On peut donc en déduire que l'autre phase, constituée d'une solution solide de polydextrose dans la copovidone, est donc elle aussi continue. Les comprimés moulés à matrice polydextrose et copovidone étant constitués de deux phases continues distinctes, leur structure physique est donc bicontinue.

### Exemple 6 : comprimé moulé à matrice polymère polydextrose et copovidone (20 : 80) comprenant 12,41 % en poids du principe actif fumarate de 2-butyl-3-[4-[3-(dibutylamino)propyl]benzoyl]-1-benzofurane-5-carboxylate d'isopropyle

On prépare un mélange physique comprenant 12,41 % en poids de principe actif fumarate de 2-butyl-3-[4-[3-(dibutylamino)propyl]benzoyl]-1-benzofurane-5-carboxylate d'isopropyle, 68,08 % en poids de copovidone commercialisé sous la dénomination Kollidon VA 64^{®} par la société BASF, 17,51 % en poids de polydextrose commercialisé par la société Danisco sous la dénomination Litesse Ultra^{®} et 2,00 % en poids de vitamine E polyéthylène glycol succinate telle que commercialisée par la société Eastman sous la dénomination Eastman^{®} Vitamin E TPGS. Le mélange physique est réalisé à une température d'environ 50°C à l'aide d'un mélangeur Rayneri à pale en hélice inversée, pendant environ 15 minutes, pour obtenir un mélange physique homogène.

On alimente une presse à injecter de la société Erinca, modèle Sprinter 11, (injection moulage) avec ce mélange. Les paramètres opératoires sont les suivants :
- température de fourreau de la première zone de chauffe : 130 °C ;
- température de fourreau de la deuxième zone de chauffe : 140 °C ;
- température de la buse : 140 °C ;
- température du canal chaud : 140 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose et copovidone ainsi obtenus (rapport pondéral polydextrose : copovidone de 20 : 80) présentent une masse moyenne de 969 mg, chaque comprimé moulé contenant une dose d'environ 100 mg du principe actif fumarate de 2-butyl-3-[4-[3-(dibutylamino)propyl]benzoyl]-1-benzofurane-5-carboxylate d'isopropyle.

Une analyse enthalpique différentielle (une seule température de transition vitreuse, égale à 86 °C) ainsi qu'une étude de diffraction aux rayons X permettent de conclure que le principe actif fumarate de 2-butyl-3-[4-[3-(dibutylamino)propyl]benzoyl]-1-benzofurane-5-carboxylate d'isopropyle n'est pas sous forme cristallisée dans cette composition (c'est-à-dire non détecté sous forme cristallisée).

### Exemple 7 : comprimé moulé à matrice polymère polydextrose et copovidone (20 : 80) comprenant 10,03 % en poids du principe actif 7-Chloro-N,N,5-triméthyl-4-oxo-3-phényl-3,5-dihydro-4H-pyridazino[4,5-b]indole-1-acétamide

On prépare un mélange physique comprenant 10,03 % en poids de principe actif 7-Chloro-*N,N,*5-triméthyl-4-oxo-3-phényl-3,5-dihydro-4*H-*pyridazino[4,5-*b*]indole-1-acétamide, 71,97 % en poids de copovidone commercialisé sous la dénomination Kollidon VA 64^{®} par la société BASF et 17,99 % en poids de polydextrose commercialisé par la société Danisco sous la dénomination Litesse Ultra^{®}. Le mélange physique est réalisé à température ambiante (environ 25 °C) à l'aide d'un mélangeur Turbula^{®}, pendant 40 minutes, pour obtenir un mélange physique homogène.

On alimente une presse à injecter de la société Erinca, modèle Sprinter 11, (injection moulage) avec ce mélange physique. Les paramètres opératoires sont les suivants :
- température de fourreau de la première zone de chauffe : 150 °C ;
- température de fourreau de la deuxième zone de chauffe : 170 °C ;
- température de la buse : 180 °C ;
- température du canal chaud : 180 °C.

Le moule utilisé est tel qu'il permet d'obtenir un comprimé moulé de taille et de forme sensiblement identiques à celles d'une gélule de taille 0.

Après refroidissement à température ambiante, les comprimés moulés à matrice polymère polydextrose et copovidone ainsi obtenus (rapport pondéral polydextrose : copovidone de 20 : 80) présentent une masse moyenne de 985 mg, chaque comprimé moulé contenant une dose d'environ 100 mg du principe actif 7-Chloro-*N,N,*5-triméthyl-4-oxo-3-phényl-3,5-dihydro-4*H*-pyridazino[4,5-*b*]indole-1-acétamide.

Une analyse enthalpique différentielle (une seule température de transition vitreuse, à 99 °C) ainsi qu'une étude de diffraction aux rayons X permettent de constater que le principe actif 7-Chloro-*N,N,*5-triméthyl-4-oxo-3-phényl-3,5-dihydro-4*H-*pyridazino[4,5-*b*]indole-1-acétamide n'est pas sous forme cristallisée dans cette composition (c'est-à-dire non détecté sous forme cristallisée).

## Revendications

1. Composition pharmaceutique solide comprenant une dispersion solide comprenant au moins un principe actif et une matrice polymère pharmaceutiquement acceptable, **caractérisée en ce que** ladite matrice polymère pharmaceutiquement acceptable comprend (i) du polydextrose, sous forme d'une phase continue de polydextrose, en mélange avec (ii) au moins un polymère autre que du polydextrose, sous forme d'une phase continue de ce polymère, la proportion dudit polydextrose étant d'au moins 20 % en poids et la proportion dudit au moins un polymère autre que du polydextrose étant d'au moins 20 % en poids, par rapport au poids total de ladite matrice polymère pharmaceutiquement acceptable.

2. Composition pharmaceutique selon la revendication 1, **caractérisée en ce qu'**elle est susceptible d'être obtenue par un procédé comprenant au moins une étape consistant à réaliser un mélange comprenant ledit au moins un principe actif, ledit polydextrose et ledit au moins un polymère autre que du polydextrose, dans un dispositif à vis mélangeuse et à une température de mélange comprise entre environ 50 °C et environ 250 °C.

3. Composition pharmaceutique selon la revendication 1 ou 2, comprenant une dispersion solide comprenant au moins un principe actif et une matrice polymère pharmaceutiquement acceptable,
**caractérisée en ce que** ladite matrice polymère pharmaceutiquement acceptable comprend (i) du polydextrose pour favoriser la désagrégation de la composition en milieu aqueux, sous forme d'une phase continue de polydextrose, en mélange avec (ii) au moins un polymère autre que du polydextrose, sous forme d'une phase continue de ce polymère, la proportion dudit polydextrose étant d'au moins 20 % en poids et la proportion dudit au moins un polymère autre que du polydextrose étant d'au moins 20 % en poids, par rapport au poids total de ladite matrice polymère pharmaceutiquement acceptable,
et **en ce que** cette composition pharmaceutique est susceptible d'être obtenue par un procédé comprenant au moins une étape consistant à réaliser un mélange comprenant ledit au moins un principe actif, ledit polydextrose et ledit au moins un polymère autre que du polydextrose, dans un dispositif à vis mélangeuse et à une température de mélange comprise entre environ 50 °C et environ 250 °C.

4. Composition pharmaceutique selon l'une quelconque des revendications précédentes, **caractérisée en ce que** ledit polydextrose est choisi dans le groupe constitué par les polydextroses pharmaceutiquement acceptables présentant un poids moléculaire d'au plus 22000 g/mol, et les mélanges de ceux-ci.

5. Composition pharmaceutique selon l'une quelconque des revendications précédentes, **caractérisée en ce que** ledit au moins un polymère autre que du polydextrose est choisi dans le groupe constitué par les polymères cellulosiques, les homo- et copolymères vinyliques, les polymères acryliques et méthacryliques, les amidons chimiquement modifiés, les pectines, les dérivés de la chitine, la gomme adragante, la gélatine, l'alginate de sodium, le pullulane, la gomme arabique, la gomme guar, l'agar-agar, la gomme xanthane, les polyakylèneoxides, et les mélanges de ceux-ci.

6. Composition pharmaceutique selon la revendication 5, **caractérisée en ce que** ledit au moins un polymère autre que du polydextrose est choisi dans le groupe constitué par la méthylcellulose, l'hydroxyméthylcellulose, l'hydroxyéthylcellulose, l'hydroxypropylcellulose, l'hydroxybutylcellulose, l'hydroxypropylcellulose faiblement substituée, l'hydroxyéthylméthylcellulose, l'hydroxypropylméthylcellulose, la carboxyméthylcellulose, la carboxyméthylcellulose sodium, la carboxyméthyléthylcellulose, l'hydroxypropylméthylcellulose phtalate, l'hydroxypropylméthylcellulose acétate succinate, la cellulose acétate phtalate, la povidone, la copovidone, le polyvinyl alcool, les polymères acryliques et méthacryliques, les amidons dérivés d'amidons extraits du maïs, de la pomme de terre, du riz, du blé ou du tapioca, les pectines, le chitosan, la gomme adragante, la gélatine, l'alginate de sodium, le pullulane, la gomme arabique, la gomme guar, l'agar-agar, la gomme xanthane, les polyéthylèneoxydes, les polypropylèneoxydes, les copolymères d'oxyde d'éthylène et d'oxyde de propylène, et les mélanges de ceux-ci.

7. Composition pharmaceutique selon l'une quelconque des revendications précédentes, **caractérisée en ce que** ledit au moins un polymère autre que du polydextrose est choisi dans le groupe constitué par les polymères hydrophiles autres que du polydextrose, et les mélanges de ceux-ci.

8. Composition pharmaceutique selon l'une quelconque des revendications précédentes, **caractérisée en ce que** ledit au moins un polymère autre que du polydextrose est un polymère hydrophile choisi dans le groupe constitué par l'hydroxypropylcellulose, l'hydroxyéthylcellulose, les copolymères cationiques de diméthylaminoéthylméthacrylates et d'esters méthacryliques neutres, les copolymères anioniques d'acide méthacrylique et d'esters d'acide méthacrylique, l'hydroxypropylméthylcellulose acétate succinate, les polyéthylène glycols, la copovidone, et les mélanges de ceux-ci.

9. Composition pharmaceutique selon l'une quelconque des revendications précédentes, **caractérisée en ce que** ledit au moins un polymère autre que du polydextrose est sous forme d'une phase continue d'une solution solide de polydextrose dans ledit au moins un polymère autre que du polydextrose.

10. Composition pharmaceutique selon l'une quelconque des revendications précédentes, **caractérisée en ce que** ladite matrice polymère pharmaceutiquement acceptable a une structure bicontinue essentiellement constituée d'une phase continue de polydextrose et d'une phase continue dudit au moins un polymère autre que du polydextrose.

11. Composition pharmaceutique selon la revendication 10, **caractérisée en ce que** ladite matrice polymère pharmaceutiquement acceptable a une structure bicontinue essentiellement constituée d'une phase continue de polydextrose et d'une phase continue dudit au moins un polymère autre que du polydextrose choisi dans le groupe constitué par les polymères hydrophiles et les mélanges de ceux-ci selon la revendication 7 ou 8.

12. Composition pharmaceutique selon l'une quelconque des revendications précédentes, **caractérisée en ce que** la proportion dudit polydextrose est comprise entre environ 20 % et environ 80 % en poids, et **en ce que** la proportion dudit au moins un polymère autre que du polydextrose est comprise entre environ 20 % et environ 80 % en poids, par rapport au poids total de la matrice polymère pharmaceutiquement acceptable.

13. Composition pharmaceutique selon l'une quelconque des revendications précédentes, **caractérisée en ce que** le rapport pondéral dudit polydextrose audit au moins un polymère autre que du polydextrose, dans ladite matrice polymère, est compris entre environ 20 : 80 et environ 50 : 50.

14. Composition pharmaceutique selon l'une quelconque des revendications précédentes, **caractérisée en ce que** la proportion de ladite matrice polymère pharmaceutiquement acceptable est comprise entre environ 50 % et environ 99,9 % en poids, par rapport au poids total de la composition.

15. Composition pharmaceutique selon l'une quelconque des revendications précédentes, **caractérisée en ce que** ledit au moins un principe actif est majoritairement à l'état amorphe dans ladite matrice polymère pharmaceutiquement acceptable.

16. Composition pharmaceutique selon l'une quelconque des revendications précédentes, **caractérisée en ce que** ledit principe actif est choisi dans le groupe constitué par :
- le N-pipéridino-5-(4-bromophényl)-1-(2,4-dichlorophényl)-4-méthylpyrazole-3-carboxamide ;
- le N-pipéridino-5-(4-chlorophényl)-1-(2,4-dichlorophényl)-4-méthylpyrazole-3-carboxamide ;
- l'amiodarone (ou 2-n-butyl-3-[3,5-diiodo-4-diéthylaminoéthoxy-benzoyl]benzofurane) et ses sels pharmaceutiquement acceptables notamment le chlorhydrate ;
- la dronédarone (ou 2-n-butyl-3-[4-(3-di-n-butylaminopropoxy)benzoyl]-5-méthylsulfonamido-benzofurane) et ses sels pharmaceutiquement acceptables notamment le chlorhydrate ;
- l'acide 2-[1-(7-chloroquinoline-4-yl)-5-(2,6-diméthoxyphenyl)-1H-pyrazole-3-carbonyl]amino-adamantane-2-carboxylique ;
- le 2-n-butyl-3-[4-[3-(dibutylamino)propyl]benzoyl]-1-benzofurane-5-carboxylate d'isopropyle et ses sels pharmaceutiquement acceptables notamment le fumarate;
- le 7-Chloro-*N,N*,5-triméthyl-4-oxo-3-phényl-3,5-dihydro-4*H*-pyridazino[4,5-b]indole-1-acétamide,
et les associations de ces principes actifs.

17. Composition pharmaceutique selon l'une quelconque des revendications précédentes, **caractérisée en ce que** la proportion dudit principe actif est comprise entre environ 0,1 % et environ 50 % en poids, par rapport au poids total de la composition.

18. Composition pharmaceutique selon l'une quelconque des revendications 2 à 17, caractérisée en ce ledit mélange réalisé dans un dispositif à vis mélangeuse comprend en outre au moins un composant choisi dans le groupe constitué par les agents plastifiants, les agents de démoulage ou lubrifiants, les agents fluidifiants, les agents antioxydants, les agents conservateurs, les colorants, les arômes, les édulcorants, les agents mouillants, les agents tampons, les agents adsorbants, les agents absorbants, les promoteurs d'absorption, les agents bioadhésifs, les agents désintégrants et les mélanges de ceux-ci.

19. Composition pharmaceutique selon l'une quelconque des revendications 2 à 18, **caractérisée en ce que** ladite température de mélange est comprise entre environ 80 °C et environ 200 °C.

20. Composition pharmaceutique selon l'une quelconque des revendications 2 à 19, **caractérisée en ce que** ledit dispositif à vis mélangeuse est un dispositif à bi-vis mélangeuse.

21. Composition pharmaceutique selon l'une quelconque des revendications 2 à 20, **caractérisée en ce que** ledit dispositif à vis mélangeuse est un dispositif pour extrusion.

22. Composition pharmaceutique selon la revendication 21, **caractérisée en ce que** ladite l'étape consistant à réaliser ledit mélange dans un dispositif pour extrusion est suivie d'au moins une étape consistant à mettre en forme le mélange extrudé, à la température du mélange extrudé ou après refroidissement du mélange extrudé à une température de mise en forme appropriée, choisie dans le groupe constitué par les étapes de calandrage, de filage, de découpage, et les combinaisons de ces étapes.

23. Composition pharmaceutique selon l'une quelconque des revendications 2 à 20, **caractérisée en ce que** ledit dispositif à vis mélangeuse est un dispositif pour injection moulage.

24. Composition pharmaceutique selon l'une quelconque des revendications 2 à 23, **caractérisée en ce qu'**elle est susceptible d'être obtenue par un procédé comprenant en outre, après refroidissement à une température appropriée pour solidifier de manière suffisante le mélange obtenu, au moins une étape choisie dans le groupe constitué par les étapes de broyage, de découpage, et les combinaisons de ces étapes.

25. Composition pharmaceutique selon l'une quelconque des revendications 2 à 24 précédentes, **caractérisée en ce qu'**elle est susceptible d'être obtenue par un procédé comprenant en outre au moins une étape d'enrobage pour une libération modifiée.

26. Forme pharmaceutique solide, **caractérisée en ce qu'**elle comprend au moins une composition pharmaceutique selon l'une quelconque des revendications précédentes.

27. Comprimé pharmaceutique, **caractérisé en ce qu'**il est susceptible d'être obtenu par un procédé comprenant au moins une étape de compression ou de compactage d'au moins une composition pharmaceutique selon la revendication 24.

28. Comprimé pharmaceutique selon la revendication 27, **caractérisé en ce qu'**il est susceptible d'être obtenu par un procédé comprenant en outre au moins une étape d'enrobage pour une libération modifiée.

29. Gélule pharmaceutique, **caractérisée en ce qu'**elle est susceptible d'être obtenue par un procédé comprenant au moins une étape de remplissage avec au moins une composition pharmaceutique selon la revendication 24.

30. Gélule pharmaceutique, **caractérisée en ce qu'**elle est susceptible d'être obtenue par un procédé comprenant au moins une étape de remplissage avec au moins une composition pharmaceutique selon les revendications 24 et 25.

31. Comprimé pharmaceutique moulé, **caractérisé en ce qu'**il est constitué d'une composition pharmaceutique selon la revendication 23.

32. Comprimé pharmaceutique moulé, **caractérisé en ce qu'**il est constitué d'une composition pharmaceutique selon les revendications 23 et 25.

33. Utilisation du polydextrose pour la fabrication par extrusion ou injection moulage d'une composition pharmaceutique comprenant une dispersion solide d'au moins un principe actif dans une matrice polymère pharmaceutiquement acceptable, ladite matrice polymère comprenant du polydextrose, sous forme d'une phase continue de polydextrose, en mélange avec au moins un polymère autre que du polydextrose, sous forme d'une phase continue de ce polymère, la proportion dudit polydextrose étant d'au moins 20 % en poids et la proportion dudit au moins un polymère autre que du polydextrose étant d'au moins 20 % en poids, par rapport au poids total de ladite matrice polymère.

## Patentansprüche

1. Feste pharmazeutische Zusammensetzung, umfassend eine feste Dispersion, die mindestens einen Wirkstoff und eine pharmazeutisch unbedenkliche Polymermatrix umfasst, **dadurch gekennzeichnet, dass** die pharmazeutisch unbedenkliche Polymermatrix (i) Polydextrose in Form einer kontinuierlichen Polydextrosephase in Abmischung mit (ii) mindestens einem von Polydextrose verschiedenen Polymer in Form einer kontinuierlichen Phase dieses Polymers umfasst, wobei der Anteil der Polydextrose mindestens 20 Gew.-% beträgt und der Anteil des mindestens einen von Polydextrose verschiedenen Polymers mindestens 20 Gew.-% beträgt, bezogen auf das Gesamtgewicht der pharmazeutisch unbedenklichen Polymermatrix.

2. Pharmazeutische Zusammensetzung nach Anspruch 1, **dadurch gekennzeichnet, dass** sie durch ein Verfahren erhältlich ist, das mindestens einen Schritt umfasst, der darin besteht, dass man in einer Schneckenmischvorrichtung bei einer Mischtemperatur zwischen ungefähr 50° C und ungefähr 250° C eine den mindestens einen Wirkstoff, die Polydextrose und das mindestens eine von Polydextrose verschiedene Polymer umfassende Mischung herstellt.

3. Pharmazeutische Zusammensetzung nach Anspruch 1 oder 2, umfassend eine feste Dispersion, die mindestens einen Wirkstoff und eine pharmazeutisch unbedenkliche Polymermatrix umfasst,
**dadurch gekennzeichnet, dass** die pharmazeutisch unbedenkliche Polymermatrix (i) Polydextrose zur Förderung des Zerfalls der Zusammensetzung in wässrigem Medium in Form einer kontinuierlichen Polydextrosephase in Abmischung mit (ii) mindestens einem von Polydextrose verschiedenen Polymer in Form einer kontinuierlichen Phase dieses Polymers umfasst, wobei der Anteil der Polydextrose mindestens 20 Gew.-% beträgt und der Anteil des mindestens einen von Polydextrose verschiedenen Polymers mindestens 20 Gew.-% beträgt, bezogen auf das Gesamtgewicht der pharmazeutisch unbedenklichen Polymermatrix,
und dass diese pharmazeutische Zusammensetzung durch ein Verfahren erhältlich ist, das mindestens einen Schritt umfasst, der darin besteht, dass man in einer Schneckenmischvorrichtung bei einer Mischtemperatur zwischen ungefähr 50° C und ungefähr 250° C eine den mindestens einen Wirkstoff, die Polydextrose und das mindestens eine von Polydextrose verschiedene Polymer umfassende Mischung herstellt.

4. Pharmazeutische Zusammensetzung nach einem der vorhergehenden Ansprüche, **dadurch gekennzeichnet, dass** die Polydextrose aus der Gruppe bestehend aus pharmazeutisch unbedenklichen Polydextrosen mit einem Molekulargewicht von höchstens 22.000 g/mol und Mischungen davon ausgewählt ist.

5. Pharmazeutische Zusammensetzung nach einem der vorhergehenden Ansprüche, **dadurch gekennzeichnet, dass** das mindestens eine von Polydextrose verschiedene Polymer aus der Gruppe bestehend aus Cellulosepolymeren, Vinylhomopolymer und -copoly-meren, Acryl- und Methacrylpolymeren, chemisch modifizierten Stärken, Pektinen, Chitinderivaten, Tragant, Gelatine, Natriumalginat, Pullulan, Gummi arabicum, Guar-Gummi, Agar-Agar, Xanthan, Poly-alkylenoxiden und Mischungen davon ausgewählt ist.

6. Pharmazeutische Zusammensetzung nach Anspruch 5, **dadurch gekennzeichnet, dass** das mindestens eine von Polydextrose verschiedene Polymer aus der Gruppe bestehend aus Methylcellulose, Hydroxymethylcellulose, Hydroxyethylcellulose, Hydroxypropylcellulose, Hydroxybutylcellulose, schwach substituierter Hydroxypropylcellulose, Hydroxyethylmethylcellulose, Hydroxypropylmethylcellulose, Carboxymethylcellulose, Natriumcarboxymethylcellulose, Carboxymethylethylcellulose, Hydroxypropylmethylcellulosephthalat, Hydroxypropylmethylcelluloseacetatsuccinat, Cellulose-acetatphthalat, Povidon, Copovidon, Polyvinyl-alkohol, Acryl- und Methacrylpolymeren, Stärken, die sich von aus Mais, Kartoffeln, Reis, Weizen oder Tapioka extrahierten Stärken ableiten, Pektinen, Chitosan, Tragant, Gelatine, Natrium-alginat, Pullulan, Gummi arabicum, Guar-Gummi, Agar-Agar, Xanthan, Polyethylenoxiden, Polypropylenoxiden, Copolymeren von Ethylenoxid und Propylenoxid und Mischungen davon ausgewählt ist.

7. Pharmazeutische Zusammensetzung nach einem der vorhergehenden Ansprüche, **dadurch gekennzeichnet, dass** das mindestens eine von Polydextrose verschiedene Polymer aus der Gruppe bestehend aus von Polydextrose verschiedenen hydrophilen Polymeren und Mischungen davon ausgewählt ist.

8. Pharmazeutische Zusammensetzung nach einem der vorhergehenden Ansprüche, **dadurch gekennzeichnet, dass** es sich bei dem mindestens einen von Polydextrose verschiedenen Polymer um ein hydrophiles Polymer aus der Gruppe bestehend aus Hydroxypropylcellulose, Hydroxyethylcellulose, kationischen Copolymeren von Dimethylaminoethylmethacrylaten und neutralen Methacrylsäureestern, anionischen Copolymeren von Methacrylsäure und Methacrylsäureestern, Hydroxypropylmethylcelluloseacetatsuccinat, Polyethylenglykolen, Copovidon und Mischungen davon handelt.

9. Pharmazeutische Zusammensetzung nach einem der vorhergehenden Ansprüche, **dadurch gekennzeichnet, dass** das mindestens eine von Polydextrose verschiedene Polymer in Form einer kontinuierlichen Phase einer festen Lösung von Polydextrose in dem mindestens einen von Polydextrose verschiedenen Polymer vorliegt.

10. Pharmazeutische Zusammensetzung nach einem der vorhergehenden Ansprüche, **dadurch gekennzeichnet, dass** die pharmazeutisch unbedenkliche Polymermatrix eine im Wesentlichen bikontinuierliche Struktur, die aus einer kontinuierlichen Polydextrosephase und einer kontinuierlichen Phase des mindestens einen von Polydextrose verschiedenen Polymers besteht, aufweist.

11. Pharmazeutische Zusammensetzung nach Anspruch 10, **dadurch gekennzeichnet, dass** die pharmazeutisch unbedenkliche Polymermatrix eine im Wesentlichen bikontinuierliche Struktur, die aus einer kontinuierlichen Polydextrosephase und einer kontinuierlichen Phase des mindestens einen von Polydextrose verschiedenen Polymers aus der Gruppe bestehend aus den hydrophilen Polymeren und Mischungen davon gemäß Anspruch 7 oder 8 besteht, aufweist.

12. Pharmazeutische Zusammensetzung nach einem der vorhergehenden Ansprüche, **dadurch gekennzeichnet, dass** der Anteil der Polydextrose zwischen ungefähr 20 und ungefähr 80 Gew.-% liegt und dass der Anteil des mindestens einen von Polydextrose verschiedenen Polymers zwischen ungefähr 20 und ungefähr 80 Gew.-% liegt, bezogen auf das Gesamtgewicht der pharmazeutisch unbedenklichen Polymermatrix.

13. Pharmazeutische Zusammensetzung nach einem der vorhergehenden Ansprüche, **dadurch gekennzeichnet, dass** das Gewichtsverhältnis von Polydextrose zu dem mindestens einen von Polydextrose verschiedenen Polymer in der Polymermatrix zwischen ungefähr 20:80 und ungefähr 50:50 liegt.

14. Pharmazeutische Zusammensetzung nach einem der vorhergehenden Ansprüche, **dadurch gekennzeichnet, dass** der Anteil der pharmazeutisch unbedenklichen Polymermatrix zwischen ungefähr 50 und ungefähr 99,9 Gew.-%, bezogen auf das Gesamtgewicht der Zusammensetzung, beträgt.

15. Pharmazeutische Zusammensetzung nach einem der vorhergehenden Ansprüche, **dadurch gekennzeichnet, dass** der mindestens eine Wirkstoff in der pharmazeutisch unbedenklichen Polymermatrix überwiegend in amorphem Zustand vorliegt.

16. Pharmazeutische Zusammensetzung nach einem der vorhergehenden Ansprüche, **dadurch gekennzeichnet, dass** der Wirkstoff aus der Gruppe bestehend aus
- N-Piperidino-5-(4-bromphenyl)-1-(2,4-dichlorphenyl)-4-methylpyrazol-3-carboxamid;
- N-Piperidino-5-(4-chlorphenyl)-1-(2,4-dichlorphenyl)-4-methylpyrazol-3-carboxamid;
- Amiodaron (oder 2-n-Butyl-3-[3,5-diiod-4-di-ethylaminoethoxy-benzoyl]benzofuran) und pharmazeutisch unbedenklichen Salzen davon, insbesondere dem Hydrochlorid;
- Dronedaron (oder 2-n-Butyl-3-[4-(3-di-n-butyl-aminopropoxy)benzoyl]-5-methylsulfonamidobenzo-furan) und pharmazeutisch unbedenklichen Salzen davon, insbesondere dem Hydrochlorid;
- 2-[1-(7-Chlorchinolin-4-yl)-5-(2,6-dimethoxy-phenyl)-1H-pyrazol-3-carbonyl]amino-adamantan-2-carbonsäure;
- 2-n-Butyl-3-[4-[3-(dibutylamino)propyl]-benzoyl]-1-benzofuran-5-carbonsäureisopropyl-ester und pharmazeutisch unbedenklichen Salzen davon, insbesondere dem Fumarat;
- 7-Chlor-N,N,5-trimethyl-4-oxo-3-phenyl-3,5-dihydro-4*H*-pyridazino[4,5-b]indol-1-acetamid und Kombinationen dieser Wirkstoffe ausgewählt ist.

17. Pharmazeutische Zusammensetzung nach einem der vorhergehenden Ansprüche, **dadurch gekennzeichnet, dass** der Anteil des Wirkstoffs zwischen ungefähr 0,1 und ungefähr 50 Gew.-%, bezogen auf das Gesamtgewicht der Zusammensetzung, beträgt.

18. Pharmazeutische Zusammensetzung nach einem der Ansprüche 2 bis 17, **dadurch gekennzeichnet, dass** die in einer Schneckenmischvorrichtung hergestellte Mischung außerdem mindestens eine Komponente umfasst, die aus der Gruppe bestehend aus Weichmachern, Entformungsmitteln oder Schmiermitteln, Fließmitteln, Antioxidantien, Konservie-rungsmitteln, Farbmitteln, Aromen, Süßstoffen, Netzmitteln, Puffersubstanzen, Adsorptionsmitteln, Absorptionsmitteln, Resorptionsverbesserern, Bio-adhäsiva, Sprengmitteln und Mischungen davon ausgewählt ist.

19. Pharmazeutische Zusammensetzung nach einem der Ansprüche 2 bis 18, **dadurch gekennzeichnet, dass** die Mischtemperatur zwischen ungefähr 80° C und ungefähr 200°C liegt.

20. Pharmazeutische Zusammensetzung nach einem der Ansprüche 2 bis 19, **dadurch gekennzeichnet, dass** es sich bei der Schneckenmischvorrichtung um eine Zweischneckenmischvorrichtung handelt.

21. Pharmazeutische Zusammensetzung nach einem der Ansprüche 2 bis 20, **dadurch gekennzeichnet, dass** es sich bei der Schneckenmischvorrichtung um eine Extrusionsvorrichtung handelt.

22. Pharmazeutische Zusammensetzung nach Anspruch 21, **dadurch gekennzeichnet, dass** der Schritt, der in der Herstellung der Mischung in einer Extrusionsvorrichtung besteht, von mindestens einem Schritt gefolgt wird, der darin besteht, dass die extrudierte Mischung bei der Temperatur der extrudierten Mischung oder nach Abkühlen der extrudierten Mischung auf eine geeignete Formungstemperatur geformt wird, ausgewählt aus der Gruppe bestehend aus Kalandrier-, Spinn- und Schneideschritten und Kombinationen dieser Schritte.

23. Pharmazeutische Zusammensetzung nach einem der Ansprüche 2 bis 20, **dadurch gekennzeichnet, dass** es sich bei der Schneckenmischvorrichtung um eine Spritzgussvorrichtung handelt.

24. Pharmazeutische Zusammensetzung nach einem der Ansprüche 2 bis 23, **dadurch gekennzeichnet, dass** sie durch ein Verfahren erhältlich ist, das außerdem nach dem Abkühlen auf eine zur ausreichenden Verfestigung der erhaltenen Mischung geeignete Temperatur mindestens einen Schritt aus der Gruppe bestehend aus Zerkleinerungs- und Schneideschritten und Kombinationen dieser Schritte umfasst.

25. Pharmazeutische Zusammensetzung nach einem der vorhergehenden Ansprüche 2 bis 24, **dadurch gekennzeichnet, dass** sie durch ein Verfahren erhältlich ist, das außerdem mindestens einen Schritt des Beschichtens zur modifizierten Freisetzung umfasst.

26. Feste pharmazeutische Form, **dadurch gekennzeichnet, dass** sie mindestens eine pharmazeutische Zusammensetzung nach einem der vorhergehenden Ansprüche umfasst.

27. Pharmazeutische Tablette, **dadurch gekennzeichnet, dass** sie durch ein Verfahren erhältlich ist, das mindestens einen Schritt der Verpressung oder Kompaktierung mindestens einer pharmazeutischen Zusammensetzung nach Anspruch 24 umfasst.

28. Pharmazeutische Tablette nach Anspruch 27, **dadurch gekennzeichnet, dass** sie durch ein Verfahren erhältlich ist, das außerdem mindestens einen Schritt des Beschichtens zur modifizierten Freisetzung umfasst.

29. Pharmazeutische Gelatinekapsel, **dadurch gekennzeichnet**, das sie nach einem Verfahren erhältlich ist, das mindestens einen Schritt des Füllens mit mindestens einer pharmazeutischen Zusammensetzung nach Anspruch 24 umfasst.

30. Pharmazeutische Gelatinekapsel, **dadurch gekennzeichnet**, das sie nach einem Verfahren erhältlich ist, das mindestens einen Schritt des Füllens mit mindestens einer pharmazeutischen Zusammensetzung nach den Ansprüchen 24 und 25 umfasst.

31. Geformte pharmazeutische Tablette, **dadurch gekennzeichnet, dass** sie aus einer pharmazeutischen Zusammensetzung nach Anspruch 23 besteht.

32. Geformte pharmazeutische Tablette, **dadurch gekennzeichnet, dass** sie aus einer pharmazeutischen Zusammensetzung nach den Ansprüchen 23 und 25 besteht.

33. Verwendung von Polydextrose zur Herstellung einer pharmazeutischen Zusammensetzung durch Extrusion oder Spritzguss, wobei die pharmazeutische Zusammensetzung eine feste Dispersion von mindestens einem Wirkstoff in einer pharmazeutisch unbedenklichen Polymermatrix umfasst, wobei die Polymermatrix Polydextrose in Form einer kontinuierlichen Polydextrosephase in Abmischung mit mindestens einem von Polydextrose verschiedenen Polymer in Form einer kontinuierlichen Phase dieses Polymers umfasst, wobei der Anteil der Polydextrose mindestens 20 Gew.-% beträgt und der Anteil des mindestens einen von Polydextrose verschiedenen Polymers mindestens 20 Gew.-% beträgt, bezogen auf das Gesamtgewicht der Polymermatrix.

## Claims

1. Solid pharmaceutical composition comprising a solid dispersion containing at least one active principle and a pharmaceutically acceptable polymer matrix, **characterized in that** said pharmaceutically acceptable polymer matrix comprises a blend of (i) polydextrose, in the form of a continuous polydextrose phase, and (ii) at least one polymer other than polydextrose, in the form of a continuous phase of this polymer, the proportion of said polydextrose being at least 20% by weight and the proportion of said at least one polymer other than polydextrose being at least 20% by weight, relative to the total weight of said pharmaceutically acceptable polymer matrix.

2. Pharmaceutical composition according to Claim 1, **characterized in that** it can be obtained by means of a process comprising at least one step consisting in producing a compound containing said at least one active principle, said polydextrose and said at least one polymer other than polydextrose, in a screw mixer and at a mixing temperature of between approximately 50°C and approximately 250°C.

3. Pharmaceutical composition according to Claim 1 or 2, comprising a solid dispersion containing at least one active principle and a pharmaceutically acceptable polymer matrix,
**characterized in that** said pharmaceutically acceptable polymer matrix comprises a blend of (i) polydextrose, in the form of a continuous polydextrose phase, in order to promote the disintegration of the composition in an aqueous medium, and (ii) at least one polymer other than polydextrose, in the form of a continuous phase of this polymer, the proportion of said polydextrose being at least 20% by weight and the proportion of said at least one polymer other than polydextrose being at least 20% by weight, relative to the total weight of said pharmaceutically acceptable polymer matrix,
and **in that** this pharmaceutical composition can be obtained by means of a process comprising at least one step consisting in producing a compound containing said at least one active principle, said polydextrose and said at least one polymer other than polydextrose, in a screw mixer and at a mixing temperature of between approximately 50°C and approximately 250°C.

4. Pharmaceutical composition according to any one of the preceding claims, **characterized in that** said polydextrose is selected from the group consisting of pharmaceutically acceptable polydextroses having a molecular weight of at most 22 000 g/mol, and blends thereof.

5. Pharmaceutical composition according to any one of the preceding claims, **characterized in that** said at least one polymer other than polydextrose is selected from the group consisting of cellulose-based polymers, vinyl homo- and copolymers, acrylic and methacrylic polymers, chemically modified starches, pectins, chitin derivatives, gum tragacanth, gelatin, sodium alginate, pullulan, gum arabic, guar gum, agar-agar, xanthan gum, polyalkylene oxides, and blends thereof.

6. Pharmaceutical composition according to Claim 5, **characterized in that** said at least one polymer other than polydextrose is selected from the group consisting of methylcellulose, hydroxymethylcellulose, hydroxyethylcellulose, hydroxypropylcellulose, hydroxybutylcellulose, weakly substituted hydroxypropylcellulose, hydroxyethylmethylcellulose, hydroxypropylmethylcellulose, carboxymethylcellulose, sodium carboxymethylcellulose, carboxymethylethylcellulose, hydroxypropylmethylcellulose phthalate, hydroxypropylmethylcellulose acetate succinate, cellulose acetate phthalate, povidone, copovidone, polyvinyl alcohol, acrylic and methacrylic polymers, starches derived from starches extracted from maize, from potato, from rice, from wheat or from tapioca, pectins, chitosan, gum tragacanth, gelatin, sodium alginate, pullulan, gum arabic, guar gum, agar-agar, xanthan gum, polyethylene oxides, polypropylene oxides, copolymers of ethylene oxide and of propylene oxide, and blends thereof.

7. Pharmaceutical composition according to any one of the preceding claims, **characterized in that** said at least one polymer other than polydextrose is selected from the group consisting of hydrophilic polymers other than polydextrose, and blends thereof.

8. Pharmaceutical composition according to any one of the preceding claims, **characterized in that** said at least one polymer other than polydextrose is a hydrophilic polymer selected from the group consisting of hydroxypropylcellulose, hydroxyethylcellulose, cationic copolymers of dimethylaminoethyl methacrylates and of neutral methacrylic esters, anionic copolymers of methacrylic acid and of methacrylic acid esters, hydroxypropylmethylcellulose acetate succinate, polyethylene glycols, copovidone, and blends thereof.

9. Pharmaceutical composition according to any one of the preceding claims, **characterized in that** said at least one polymer other than polydextrose is in the form of a continuous phase of a solid solution of polydextrose in said at least one polymer other than polydextrose.

10. Pharmaceutical composition according to any one of the preceding claims, **characterized in that** said pharmaceutically acceptable polymer matrix has a bicontinuous structure essentially consisting of a continuous polydextrose phase and of a continuous phase of said at least one polymer other than polydextrose.

11. Pharmaceutical composition according to Claim 10, **characterized in that** said pharmaceutically acceptable polymer matrix has a bicontinuous structure essentially consisting of a continuous polydextrose phase and of a continuous phase of said at least one polymer other than polydextrose selected from the group consisting of hydrophilic polymers and blends thereof according to Claim 7 or 8.

12. Pharmaceutical composition according to any one of the preceding claims, **characterized in that** the proportion of said polydextrose is between approximately 20% and approximately 80% by weight, and **in that** the proportion of said at least one polymer other than polydextrose is between approximately 20% and approximately 80% by weight, relative to the total weight of the pharmaceutically acceptable polymer matrix.

13. Pharmaceutical composition according to any one of the preceding claims, **characterized in that** the weight ratio of said polydextrose to said at least one polymer other than polydextrose, in said polymer matrix, is between approximately 20:80 and approximately 50:50.

14. Pharmaceutical composition according to any one of the preceding claims, **characterized in that** the proportion of said pharmaceutically acceptable polymer matrix is between approximately 50% and approximately 99.9% by weight, relative to the total weight of the composition.

15. Pharmaceutical composition according to any one of the preceding claims, **characterized in that** said at least one active principle is predominantly in the amorphous state in said pharmaceutically acceptable polymer matrix.

16. Pharmaceutical composition according to any one of the preceding claims, **characterized in that** said active principle is selected from the group consisting of:
- N-piperidino-5-(4-bromophenyl)-1-(2,4-dichlorophenyl)-4-methylpyrazole-3-carboxamide;
- N-piperidino-5-(4-chlorophenyl)-1-(2,4-dichlorophenyl)-4-methylpyrazole-3-carboxamide;
- amiodarone (or 2-n-butyl-3-[3,5-diiodo-4-diethylaminoethoxy-benzoyl]benzofuran) or pharmaceutically acceptable salts thereof, in particular the hydrochloride;
- dronedarone (or 2-n-butyl-3-[4-(3-di-n-butylaminopropoxy)benzoyl]-5-methylsulfonamidobenzofuran) and pharmaceutically acceptable salts thereof, in particular the hydrochloride;
- 2-[1-(7-chloroquinolin-4-yl)-5-(2,6-dimethoxyphenyl)-1H-pyrazole-3-carbonyl]amino-adamantane-2-carboxylic acid;
- isopropyl 2-n-butyl-3-[4-[3-(dibutylamino)propyl]benzoyl]-1-benzofuran-5-carboxylate and pharmaceutically acceptable salts thereof, in particular the fumarate;
- 7-chloro-N,N,5-trimethyl-4-oxo-3-phenyl-3,5-dihydro-4*H*-pyridazino[4,5-b]indol-1-acetamide,
and combinations of these active principles.

17. Pharmaceutical composition according to any one of the preceding claims, **characterized in that** the proportion of said active principle is between approximately 0.1% and approximately 50% by weight, relative to the total weight of the composition.

18. Pharmaceutical composition according to any one of Claims 2 to 17, **characterized in that** said compound produced in a screw mixer also contains at least one component selected from the group consisting of plasticizers, demolding agents or lubricants, fluidifying agents, antioxidants, preserving agents, dyes, flavorings, sweeteners, wetting agents, buffers, adsorbents, absorbents, absorption promoters, bioadhesive agents, disintegrating agents and mixtures thereof.

19. Pharmaceutical composition according to any one of Claims 2 to 18, **characterized in that** said mixing temperature is between approximately 80°C and approximately 200°C.

20. Pharmaceutical composition according to any one of Claims 2 to 19, **characterized in that** said screw mixer is a twin-screw mixer.

21. Pharmaceutical composition according to any one of Claims 2 to 20, **characterized in that** said screw mixer is an extrusion device.

22. Pharmaceutical composition according to Claim 21, **characterized in that** said step consisting in producing said compound in an extrusion device is followed by at least one step consisting in forming the extruded compound, at the temperature of the extruded compound or after cooling of the extruded compound to a suitable forming temperature, selected from the group consisting of calandering, spinning and cutting steps, and combinations of these steps.

23. Pharmaceutical composition according to any one of Claims 2 to 20, **characterized in that** said screw mixer is an injection-molding device.

24. Pharmaceutical composition according to any one of Claims 2 to 23, **characterized in that** it can be obtained by means of a process also comprising, after cooling to a suitable temperature for sufficiently solidifying the compound obtained, at least one step selected from the group consisting of milling and cutting steps, and combinations of these steps.

25. Pharmaceutical composition according to any one of the preceding Claims 2 to 24, **characterized in that** it can be obtained by means of a process also comprising at least one coating step for modified release.

26. Solid pharmaceutical form, **characterized in that** it comprises at least one pharmaceutical composition according to any one of the preceding claims.

27. Pharmaceutical tablet, **characterized in that** it can be obtained by means of a process comprising at least one step consisting of compression or compacting of at least one pharmaceutical composition according to Claim 24.

28. Pharmaceutical tablet according to Claim 27, **characterized in that** it can be obtained by means of a process also comprising at least one coating step for modified release.

29. Pharmaceutical gelatin capsule, **characterized in that** it can be obtained by means of a process comprising at least one step consisting in filling with at least one pharmaceutical composition according to Claim 24.

30. Pharmaceutical gelatin capsule, **characterized in that** it can be obtained by means of a process comprising at least one step consisting in filling with at least one pharmaceutical composition according to Claims 24 and 25.

31. Molded pharmaceutical tablet, **characterized in that** it consists of a pharmaceutical composition according to Claim 23.

32. Molded pharmaceutical tablet, **characterized in that** it consists of a pharmaceutical composition according to Claims 23 and 25.

33. Use of polydextrose for the production, by extrusion or injection molding, of a pharmaceutical composition comprising a solid dispersion of at least one active principle in a pharmaceutically acceptable polymer matrix, said polymer matrix comprising a blend of polydextrose, in the form of a continuous polydextrose phase, and at least one polymer other than polydextrose, in the form of a continuous phase of this polymer, the proportion of said polydextrose being at least 20% by weight and the proportion of said at least one polymer other than polydextrose being at least 20% by weight, relative to the total weight of said polymer matrix.
